# EUROPEAN PATENT SPECIFICATION

(11) **EP 1 901 748 B1**
(45) Date of publication and mention of the grant of the patent: **08.09.2010**
(21) Application number: 05812885.1
(22) Date of filing: 09.12.2005
(51) Int. Cl.: A61K 31/519, A61K 31/695, A61P 35/00

(54) **ANGIOGENESIS INHIBITORS**
ANGIOGENESEINHIBITOREN
INHIBITEURS D'ANGIOGENESE

(30) Priority: 09.06.2005 WO PCT/CH2005/000321
(43) Date of publication of application: 26.03.2008
(73) Proprietor: Oncalis AG, 8952 Schlieren (CH)
(72) Inventor: BERSET, Catherine, CH-5210 Windisch (CH); AUDETAT, Stephan, CH-5502 Hunzenschwil (CH); TIETZ, Julia, CH-8952 Schlieren (CH); GUNDE, Tea, CH-8005 Zürich (CH); BARBERIS, Alcide, CH-8057 Zürich (CH); SCHUMACHER, Andreas c/o Solvias AG, CH-4002 Basel (CH); TRAXLER, Peter, CH-4124 Schönenbuch (CH)
(74) Representative: Grimm, Siegfried
(86) International application number: PCT/CH2005/000736
(87) International publication number: WO 2006/131003

(56) References cited:
- US-A1- 2003 018 032

## Description

### Cross References to Related Applications

This application claims the priority of the international patent application No. PCT/CH2005/00321, filed June 9, 2005.

### Technical Field

The present invention relates to angiogenesis inhibitors, in particular receptor tyrosine kinase inhibitors, and their use for the treatment of hyperproliferative diseases, angiogenesis and disorders depending on angiogenesis such as tumour forming cancers. It also relates to a method of inhibiting angiogenesis or treating a vascular anomaly in a mammal comprising administering to the mammal an amount of an Eph receptor inhibitor which is effective for inhibiting angiogenesis or for treating the vascular anomaly in the mammal.

### Background Art

US 2003/0018032 discloses bicyclic Imidazo-3-yl-amine derivatives and their use as analgetics.

Protein kinases, in particular receptor protein tyrosine kinases (RTK), are key regulators of intercellular communication that controls cell growth, proliferation, differentiation, survival and metabolism. About 20 different RTK families have been identified that share a similar structure, namely an extracellular binding site for ligands, a transmembrane region and an intracellular tyrosine kinase domain. Extracellular ligand binding induces or stabilizes receptor dimerization leading to increased RTK kinase activity. The intracellular catalytic domain displays the highest level of conservation among RTKs and includes the ATP-binding site that catalyzes protein phosphorylation of e.g. cytoplasmic tyrosine residues, which serve as docking sites for Src homology 2 (SH2)-and phosphotyrosine-binding (PTB) domain-containing proteins such as Grb2, Shc, Src, Cbl or phospholipase Cγ. These proteins subsequently recruit additional effectors containing SH2, SH3, PTB and pleckstrin-homology (PH) domains'to the activated receptor, which results in the assembly of signaling complexes at the membrane and the activation of a cascade of intracellular biochemical signals. The most important downstream signaling cascades activated by RTKs include the Ras-extracellular regulated kinase (ERK)-mitogen activated (MAP) kinase pathway, the phosphoinositide 3-kinase (PI 3-kinase)-Akt and the JAK/STAT pathway. The complex signaling network triggered by RTKs eventually leads either to activation or repression of various subsets of genes and thus defines the biological response to a given signal.

The activity of RTKs and their mediated cellular signaling is precisely coordinated and tightly controlled in normal cells. Deregulation of the RTK signaling system, either by stimulation through growth factor and/or through genetic alteration, result in deregulated tyrosine kinase activity. These aberrations generally result in RTKs with constitutive or strongly enhanced kinase activity and subsequent signaling capacity, which leads to malignant transformation. Therefore, they are frequently linked to human cancer and also to other hyperproliferative diseases such as psoriasis. The most important mechanisms leading to constitutive RTK signaling include overexpression and/or gene amplification of RTKs, genetic alterations such as deletions and mutations within the extracellular domain as well as alterations of the catalytic site, or autocrine-paracrine stimulation through aberrant growth factor loops.

For example, in many human cancers, gene amplification and/or overexpression of RTKs occurs, which might increase the response of cancer cells to normal growth factor levels. Additionally, overexpression of a specific RTK on the cell surface increases the incidence of receptor dimerization even in the absence of an activating ligand. In many cases this results in constitutive activation of the RTK leading to aberrant and uncontrolled cell proliferation and tumour formation. An important example for such a scenario is HER2, also known as ErbB2, that belongs to the epidermal growth factor (EGF) receptor family of RTKs. Overexpression of HER2 was found in various types of human cancers, especially in human breast and ovarian carcinomas. Most importantly, aberrantly elevated levels of HER2 correlate with more aggressive progression of disease and reduced patient survival time. EGFR, which was the first receptor tyrosine kinase to be molecularly cloned, also plays a fundamental role in tumorigenesis. EGFR is frequently overexpressed in non-small-cell lung, bladder, cervical, ovarian, kidney and pancreatic cancer and in squamous-cell carcinomas of the head and neck. The predominant mechanism leading to EGFR overexpression is gene amplification with up to 60 copies per cell reported in certain tumours. In general, elevated levels of EGFR expression are associated with high metastatic rate and increased tumour proliferation.

A number of endothelial cell RTKs such as VEGFR, Tie and ephrin (Eph) RTK are known to be critical mediators of angiogenesis. Angiogenesis, the formation of new blood vessels from pre-existing vasculature, is a multi-step process involving many various factors, which stimulate endothelial cell proliferation, migration, and assembly, as well as recruitment of perivascular cells and extracellular matrix remodelling.

Angiogenesis is implicated in the pathogenesis of a variety of disorders, including solid tumours, intraocular neovascular syndromes such as proliferative retinopathies or age-related macular degeneration (AMD), rheumatoid arthritis, and psoriasis.

Ephrins and their receptors were first identified as guides for neuronal growth during development.

The Eph family of receptor tyrosine kinases is the largest known family of RTKs, with 16 receptors and 9 ligands identified. Homologues of Eph and ephrins have been found in vertebrate and invertebrate species, such as mice, Xenopus laevis, zebrafish and Caenorabhditis elegans.

Unlike other families of RTKs, which bind soluble ligands, Eph receptors interact with cell surface-bound ephrin ligands. Ephrins attach to the cell membrane either through a glycosylphosphatidyl inositol (GPI) anchor (ephrinA) or a transmembrane domain (ephrinB). Eph receptors are divided in two subclasses: A and B, depending on the type of interaction with their ligands ephrinA, or B. The receptor-ligand interactions activate signaling pathways in a bi-directional fashion, through both the Eph receptors and ephrin ligands.

Ephrins and their receptors mediate cellular repulsion, adhesion, cell attachment to extracellular matrices, and cell migration in various cell types. Their functions are best studied in the nervous system: they govern proper cell migration and positioning during neural development. They are also active in other cell types and are important determinants of cell morphogenesis, tissue patterning, angiogenesis and neural plasticity. Ephrins, and their receptors have been shown to play an essential role in vascular development during embryogenesis and in adult angiogenesis, as key regulators of vascular assembly, arteriovenous differentiation, and boundary formation.

Both EphA and EphB receptors and their ligands are involved in vascular development. Especially, ephrin B2 is expressed in arterial endothelial cells, whereas its cognate receptor, EphB4 is expressed in venous endothelial cells. This makes these two molecules the best markers for arterial and venous endothelial cells at very early stage of development. Other EphB like EphB1 and EphB3 are also expressed at the sites of neovascularization. EphB2 is found in mesenchyme supporting cells. Gene knock out experiments of ephB2^{-/-}, ephB4^{-/-}, or the double knock out ephB2^{-/-}ephB3^{-/-} showed defective vessel remodelling.

Since protein kinases, in particular tyrosine kinases, have been implicated in a variety of cancer indications, RTKs and the activated signaling cascades represent promising areas for the development of target-selective anticancer drugs.

Eph receptors and ephrins are recognized potential targets in angiogenesis and cancer. For reviews see Cheng, Brantley et al. 2002; Brantley-Sieders, Parker et al. 2004; Brantley-Sieders and Chen 2004; Surawska, Ma et al. 2004; Davy and Soriano 2005; Pasquale 2005.

One approach to inhibit aberrant RTK signaling is the development of small-molecule drugs that selectively interfere with their intrinsic tyrosine kinase activity and thereby block receptor autophosphorylation and activation of downstream signal transducers.

### Disclosure of the invention

Hence, it is a general object of the present invention to provide compounds having a protein kinase inhibitory activity which can be used for the treatment of disorders involving a protein kinase, in particular a EphB-type RTK, such as hyperproliferative and angiogenesis related diseases.

Now, in order to implant this and still further objects of the invention, which become more readily apparent as the description proceeds, said protein kinease inhibitor of the formula I, 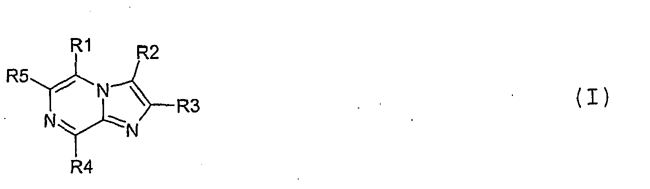 wherein R1 and R5 are independently selected from hydrogen, halogen, cyano, nitro, CF₃, optionally substituted linear or branched C₁-C₆ alkyl, optionally substituted C₁-C₆ alkenyl, optionally substituted C₁-C₆ alkinyl, optionally substituted C₃-C₈ cycloalkyl, alkoxy, NR₆R₇, OR₆, SR₆, (CH₂)ₙCHR₆R₇, wherein n is 0, 1, 2, 3, 4 and R₆ and R₇ are independently selected from hydrogen, optionally substituted C₃-C₈ cycloalkyl, optionally substituted aryl, optionally substituted 5- or 6- membered heterocycle, (CH₂)ₙC(O)OR₈, (CH₂)ₙR_{9,} wherein n is as defined above, R₈ is hydrogen, optionally substituted C₁-C₆ alkyl, optionally substituted C₃-C₈ cycloalkyl, optionally substituted aryl and R₉ is selected from hydrogen, C₁-C₆ alkoxy, optionally substituted C₃-C₈ cycloalkyl, optionally substituted aryl, halogen, hydroxyl, NO₂ NH₂, SO₂NH_{2,} cyano, and wherein all optional substituents are selected from the group of methyl, methoxy, halogen, OH, NO₂, NH₂, -C(O)OMe, cyano;

R₂ and R₄ are independently selected from hydrogen, optionally substituted linear or branched C₁-C₆ alkyl, optionally substituted C₁-C₆ alkenyl, optionally substituted C₁-C₆,alkinyl, optionally substituted C₃-C₈ cycloalkyl, NR₆R₇, OR₆, SR₆, wherein R₆ and R₇ are independently selected from hydrogen, optionally substituted C₃-C₈ cycloalkyl, optionally substituted aryl, optionally substituted 5- or 6- membered heterocycle, (CH₂)ₙC(O)OR₈, (CH₂)ₙR₉, wherein n, R₈ and R₉ are as defined above;

R₃ is hydroxyl, halogen, NH₂, NO₂, cyano, SH, optionally substituted C₁-C₆ alkyl, optionally substituted C₃-C₈ cycloalkyl, optionally substituted aryl, optionally substituted 5- or 6-membered heterocycle,
is used for the manufacture of a medicament for the treatment of a hyperproliferative and/or angiogenesis related disease.

Preferred compounds of formula I are those where R1 and R5 are independently selected from hydrogen, halogen, cyano, nitro, CF₃ and methyl, whereby hydrogen, methyl or OH are most preferred for R₅. In some more preferred compounds either R₁ or R₅ is hydrogen, or else both of them are hydrogen.

The preferences for R₂ and R₄ are such that they are independently selected from hydrogen, alkyl or NHR₇, with R₇ selected from hydrogen, optionally substituted C₃-C₈ cycloalkyl, optionally substituted aryl, optionally substituted 5- or 6- membered heterocycle, (CH₂)ₙC(O)OR₈, (CH₂)ₙR_{9,} wherein n is 0, 1, 2, 3, 4, R₈ is hydrogen, C₁-C₆ alkyl, optionally substituted C₃-C₈ cycloalkyl, optionally substituted aryl and R₉ is selected from hydrogen, C₁-C₆ alkoxy, optionally substituted C₃-C₈ cycloalkyl, optionally substituted aryl, halogen, hydroxyl, NO₂ NH₂, SO₂NH₂, cyano. More preferably R2 is NHR₇, and most preferably R₇ is an aryl or preferably a phenyl that can be substituted. From the substituted phenyls, a single halogen is preferred.

The preference for R₄ is hydrogen, methyl or NR₁₀R₁₁, which are independently selected from hydrogen, methyl or phenylmethyl.

The preference for R3 is C₁-C₆ alkyl, optionally substituted aryl or optionally substituted 5- or 6-membered heterocycle, and most preferably optionally substituted phenyl.

Particularly preferred compounds of the present invention have an optionally substituted phenyl R₃ substituent and an R₂ substituent NHR₇ with R₇ being optionally substituted phenyl.

In a second aspect the present invention relates to a compound of formula Ia 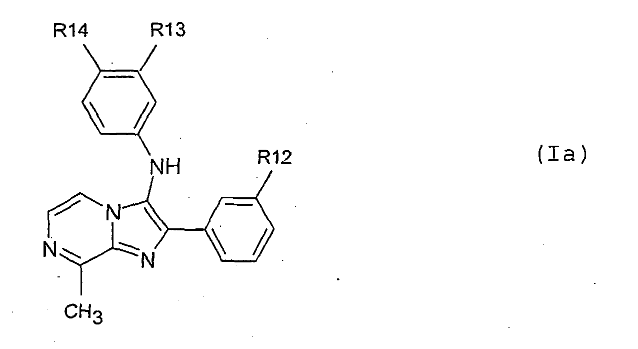 wherein R12 is hydrogen or methoxy and R13 and R14 are independently selected from hydrogen, methyl, halogen. Some of these molecules are preferred forms. R12 can be either hydrogen or methoxy, R13 can be hydrogen, methyl, or halogen that is preferably selected from Cl, F, Br, and R14 can be hydrogen or halogen that is preferably selected from Cl and F. Any combinations of R12, R13 and R14 are possible. The preferred combinations are the molecules No. 3 or No. 41-48, which are shown in Table 1 or 2, respectively.

The compounds of the present invention can be used in medical applications. Preferably, they are used for the treatment of a disease which involves a tyrosine kinase, preferably a receptor tyrosine kinase. Among the RTKs, the eph family of receptor tyrosine kinases, are particularly preferred targets for inhibition by the molecules of the present invention; among these more preferred are the EphB members and most preferred the EphB2 and EphB4 RTKs.

The compounds of the present invention can be used for the manufacture of a medicament for the treatment of many diseases involving RTKs. These diseases are hyperproliferative diseases, in particular cancer, and angiogenesis related diseases.

The compounds of the present invention can as well be used as research tools in functional genomics, drug discovery, target validation and ex vivo diagnostics.

### Modes for carrying out the invention

In the context of the present invention it has been surprisingly found that the compounds of formula I, besides the known analgetic activity of some of them (US2003/ 0018032), exhibit a tyrosine kinase inhibiting activity.

In a preferred embodiment the compounds of the present invention are directed against members of the Eph family of RTKs, preferably of the EphB type and most preferred against EphB2 or EphB4.

The compounds of the present invention are preferably used for the treatment of a hyperproliferative disease, in particular cancer. They are particularly suitable for the treatment of a hyperproliferative disorder involving a receptor tyrosine kinase of the eph family, preferably EphB2 and EphB4, or a tumour involving the cytoplasmic tyrosine kinase src. The src non-receptor tyrosine kinase is known to be involved in the development of various cancers. For a review see the publication of Warmuth et al., Curr. Pharm. Des. 2003: 9(25):2043-59.

The present invention also relates to a method of inhibiting angiogenesis or treating a vascular anomaly in a mammal comprising administering to the mammal an amount of an Eph receptor inhibitor which is effective for inhibiting angiogenesis or for treating the vascular anomaly in the mammal. The Eph familiy of RTKs are part of a signalling system that is used to regulate angiogenesis, not only as regulators of vascular remodeling during embryogenesis, but also as regulators of tumour vascularization (Brantley-Sieders and Chen, 2004; Klagsbrun M. and Eichmann, A. (2005)). Eph RTKs are also important for lymphangiogenesis (Tammela et al. 2005), an important factor in tumour metastasis and inflammation (Stacker et al. 2004). Therefore, their role in pathologic angiogenesis, particularly in cancer and inflammation, makes the Eph RTKs interesting targets for approaches in anti-angiogenic therapy.

Preferred diseases treated by administering the compounds of the present invention are psoriasis, Kaposi's sarcoma, restenosis, endometriosis, Crohn's disease, leukaemia, arthritis, rheumatoid arthritis, hemangioma, angiofibroma, diabetic retinopathy, neovascular glaucoma, renal diseases, glomerulonephritis, diabetic nephropathy, malignant nephrosclerosis, thrombotic microangiopathic syndromes, transplant rejections, glomerulopathy, cirrhosis of the liver, mesangial cell-proliferative diseases, arteriosclerosis, injuries of the nerve tissue. These diseases are triggered by (persistent) angiogenesis.

Thus, the present invention provides a method of treating diseases or disorders characterized by undesirable or excessive vascularisation, including by way of example tumours, and especially solid benign and malignant tumours, rheumatoid arthritis, psoriasis, atherosclerosis, diabetic and other retinopathies, retrolental fibroplasia, age-related macular degeneration, neovascular glaucoma, hemangiomas, thyroid hyperplasias (including Grave's disease), corneal and other tissue transplantation, and chronic inflammation, by administering an effective amount of an Eph receptor inhibitor to a patient in need thereof.

The present invention also provides a method for inhibiting the re-occlusion of vessels after balloon catheter treatment, for holding vessels open in vascular prosthetics or after inserting mechanical devices, such as, e.g., stents, as immunosuppressants, as an aid in scar-free wound healing, and for treating dermatitis.

The invention further provides a method of treating diseases or disorders characterized by undesirable or excessive vascular permeability, such as edema associated with brain tumours, ascites associated with malignancies, Meigs' syndrome, lung inflammation, nephrotic syndrome, pericardial effusion (such as that associated with pericarditis), and pleural effusion.

The Eph receptor inhibitor is further used to treat a vascular anomaly (e.g. small vessel anomalies) resulting from estrogen therapy.

The inhibitors are useful in the treatment of various neoplastic and non-neoplastic diseases and disorders. Cancers and related conditions that are amenable to treatment include breast carcinomas, lung carcinomas, gastric carcinomas, esophageal carcinomas, colorectal carcinomas, liver carcinomas, ovarian carcinomas, thecomas, arrhenoblastomas, cervical carcinomas, endometrial carcinoma, endometrial hyperplasia, endometriosis, fibrosarcomas, choriocarcinoma, head and neck cancer, nasopharyngeal carcinoma, laryngeal carcinomas, hepatoblastoma, Kaposi's sarcoma, melanoma, skin carcinomas, hemangioma, cavernous hemangioma, hemangioblastoma, pancreas carcinomas, retinoblastoma, astrocytoma, glioblastoma, Schwannoma, oligodendroglioma, medulloblastoma, neroblastomas, rhabdomyosarcoma, osteogenic sarcoma, leiomyosarcomas, urinary tract carcinomas, thyroid carcinomas, Wilm's tumour, renal cell carcinoma, prostate carcinoma, abnormal vascular proliferation associated with phakomatoses, edema (such as that associated with brain tumours), and Meigs' syndrome.

Advantageously, the compounds of the present invention are used to treat a benign or malignant neoplasia, including for example, but not exclusively, tumours of the brain, kidney, liver, adrenal gland, bladder, breast, stomach, ovaries, colon, rectum, prostate pancreas, lung, vagina, thyroid, connective tissue (sarcoma), gastrointestinal tract, tumours of the neck and head, tumours derived from cells of the hematopietic system (including leukemias, lymphomas and multiple myeloma), epidermal hyperproliferation, including for example, but not exclusively, prostate hyperplasia.

Non-neoplastic conditions that are amenable to treatment include rheumatoid arthrits, psoriasis, atherosclerosis, diabetic and other proliferative retinopathies including retinopathy of prematurity, retrolental fibroplasia, neovascular glaucoma, age-related macular degeneration, diabetic retinopathy, central retinal vein occlusion, thyroid hyperplasias (including Grave's disease), corneal and other tissue transplantation, chronic inflammation, lung inflammation nephrotic syndrome, preeclampsia, ascites, pericardial effusion (such as associated with pericarditis), and pleural effusion.

There exists evidence that receptor tyrosine kinases of the eph family are involved in the development of tumours such as breast cancer, liver cancer, gastrointestinal cancer, neuroblastomas, leukemias and lymphomas, prostate cancer, pancreatic cancer, lung cancer, melanoma, ovarian cancer, thyroid cancers, sarcoma, renal carcinomas and epidermoid cancer (M. Nakamoto et al, Microscopy Research and technique 59:58-62 (2002)). Moreover, several Eph receptors are overexpressed in various tumour types. In particular, EphB2 expression was predominantly seen in gastrointestinal and neuronal tumours in a microarray of different human tumour types (Lugli, Spichtin et al. 2005). Also, EphB4 has been implicated in breast cancer, in endometrial hyperplasias and carcinomas, small lung carcinomas, colon carcinomas, and bladder cancer (Dodelet and Pasquale 2000; Liu, Ahmad et al. 2002; Munarini, Jager et al. 2002; Berclaz, Karamitopoulou et al. 2003; Xia, Kumar et al. 2005). These tumours are preferred targets for treatment with the compounds of the present invention. However, the compounds of the present invention can be used for the treatment of all of the above mentioned types of tumours.

Eph receptors and in particular those belonging to the EphB family such as EphB4 also participate in adult hematopoiesis. Primitive human CD34+ hematopoietic cells undergo accelerated differentiation in the context of activated EphB4. Inhibitors of EphB4 could also be useful in stem cell expansion because EphB4 modifies the rate and magnitude of ES cells acquiring genotypic and phenotypic characteristics of mesodermal tissues (Wang, Miura et al. 2002; Wang, Cohen et al. 2004).

The term "protein kinase" as used herein encompasses all types of protein kinases such as serine/threonine kinases, receptor tyrosine kinases and non-receptor tyrosine kinases.

The term "angiogenesis related disease" as used herein encompasses diseases depending on or triggered by angiogenesis.

The compounds of the present invention having formula I can be prepared by methods described e.g. in WO 01/27111 or in WO 03/031447. The compounds of the present invention having formula II can be prepared by methods described e.g. in US 2003/0225098.

The compounds of the invention can be administered in a variety of dosage forms, e.g. orally, in the form of tablets, capsules, sugar- or film-coated tablets, liquid solutions or suspensions; rectally, in the form of suppositories; parenterally, e.g. intramuscularly, or by intravenous injection of infusion; or topically. The dosage depends on the age, weight, condition of the patient and administration route.

The pharmaceutical compositions containing the compounds of the invention are usually prepared following conventional methods and are administered in a pharmaceutically suitable form.

For example, the solid oral forms may contain, together with the active compound, diluents, e.g. lactose, dextrose, saccharose, cellulose, corn starch or potato starch; lubricants, e.g. silica, talc, stearic acid, magnesium or calcium stearate, and/or polyethylene glycols; binding agents, e.g. starches, arabic gums, gelatin, methylcellulose, carboxymethylcellulose or polyvinyl pyrrolidone; disaggregating agents, e.g. a starch, alginic acid, alginates or sodium starch glycolate, effervescing mixtures; dyestuffs; sweeteners; wetting agents, such as lecithin, polysorbates, laurylsulphates; and, in general, non-toxic and pharmacologically inactive substances used in pharmaceutical formulations. Said pharmaceutical preparations may be manufactured in known manner, for example by means of mixing, granulating, tabletting, sugar-coating or filmcoating processes.

The liquid dispersion for oral administration may be, e.g., syrups, emulsions and suspensions.

The syrup may contain as carrier, for example, saccharose or saccharose with glycerin and/or mannitol and/or sorbitol.

The suspensions and the emulsions may contain as carrier, for example, a natural gum, agar, sodium alginate, pectin, methylcellulose, carboxymethylcellulose or polyvinyl alcohol. The suspensions or solutions for intramuscular injections may contain, together with the active compound, a pharmaceutically acceptable carrier, e.g. sterile water, olive oil, ethyl oleate, glycols, e.g. propylene glycol, and, if desired, a suitable amount of lidocaine hydrochloride.

The solutions for intravenous injections or infusion may contain as carrier, for example, sterile water or, preferably, they may be in the form of sterile aqueous, isotonic saline solutions.

The suppositories may contain, together with the active compound, a pharmaceutically acceptable carrier, e.g. cocoa-butter, polyethylene glycol, a polyoxyethylene sorbitan fatty acid ester surfactant or lecithin.

Compositions for topical application, e.g. creams, lotions or pastes, can be prepared by admixing the active ingredient with a conventional oleaginous or emulsifying excipient.

The compounds of the present invention may be administered to a patient in form of phamaceutically acceptable salts. Suitable pharmaceutically-acceptable salts include acid addition salts such as methane-sulfonate, fumarate, hydrochloride, citrate, maleate, tartrate and hydrobromide. Also suitable are salts formed with phosphoric and sulfuric acid. Further suitable salts are base salts such as an alkali metal salt for example sodium, an alkaline earth metal salt for example calcium or magnesium, an organic amine salt for example triethylamine, morpholine, N-methylpiperidine, N-ethylpiperidine, procaine, dibenzylamine, N,N-dibenzylethylamine, tris-(2-hydroxyethyl)amine, N-methyl d-glucamine and amino acids such as lysine. There may be more than one cation or anion depending on the number of charged functions and the valency of the cations or anions.

The compounds of the present invention may be administered in the form of a pro-drug which is broken down in the human or animal body to give a compound of the present invention. A prodrug may be used to alter or improve the physical and/or pharmacokinetic profile of the parent compound and can be formed when the parent compound contains a suitable group or substituent which can be derivatised to form a prodrug. Examples of prodrugs include in-vivo hydrolysable esters of a compound of the present invention or a pharmaceutically-acceptable salt thereof.

### EXAMPLES

The invention is further illustrated by the following preparations and examples wich should not be construed to limit the scope of the disclosure. Alternative pathways and analogous structures will be apparent to those skilled in the art.

The following abbreviations are used in the following examples:
rt = room temperature (ca. 25°C)
Rt = Retention time
AcOH = acetic acid
EtOAc = ethyl acetate
EtOH = ethanol
MeOH = methanol
TFA = trifluoro acetic acid
NMR = nuclear magnetic resonance spectroscopy
HPLC = high pressure liquid chromatography
LC/MS = liquid chromatography mass spectrometry
RP = reverse phase

The following purification methods were applied to obtain pure samples: Crystallization from typical organic solvents, flash chromatography on silica gel, preparative HPLC on RP-silica gel and any combinations thereof.

For preparative HPLC, an Agilent Series 1100 Instrument with a Zorbax SB-C18 column, 21.2 x 250 mm, 7 p, was used; solvents CH₃CN - water (0.1 % TFA each).

Where HPLC data are presented, analysis was done on a Agilent Series 1100 Instrument with a Supelco Discovery C18 column (4.6 x 50 mm, 5 µ, detecting at 254 nm and 220 nm; gradient 10 % to 99 % CH₃CN within 4 minutes, 1 min. at 99 % CH₃CN using CH₃CN - water (0.1 % TFA each) solvent system with a flow rate of 2.0 mL / min. The retention times in minutes are given.

Where NMR Data are presented, ¹H spectra were obtained on a Bruker DPX 300 (300.13 MHz) and are reported as ppm down field from tetramethylsilane with the number of protons.

Where LC/MS data are presented, analysis was performed using a Micromass ZQ, (150-1000 u), ESI-positive spectrometer and Agilent Series 1100 Instrument, with a YMC-Pack ProC 18 (3 µm), 33x3 mm column; gradient flow 5 % CH₃CN /methanol/ 95% water/ 0.05% formic acid to 100% CH₃CN /methanol/ 0% water/ 0.05% formic acid within 3 minutes using CH₃CN /methanol (80:20) - water (0.05% HCOOH) as solvent system with a flow rate of 1.7 mL/min. The retention times in minutes and observed parent ion are given.

### INTERMEDIATE 1. 3-Methyl-pyrazin-2-ylamine

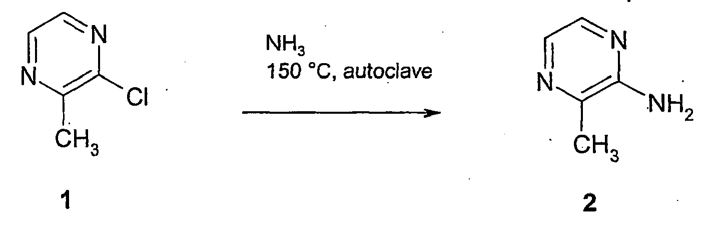

In an autoclave, 2-chloro-3-methyl-pyrazine **(1)** (10.0 g, 77.8 mmol) was dissolved in dry methanol (30 mL). Ammonia gas (60 g) was added. The mixture was heated to 150°C for 8 hours. (start pressure: 10 bar, end pressure: 90 bar). After cooling to rt, the mixture was evaporated to a brown solid, which was dissolved in 1N hydrochloric acid (100 mL) and washed with dichloromethane. The aqueous layer was slowly poured on cold saturated aqueous ammonia (150 mL), then extracted with dichloromethane (3 x 100 mL). The combined organic layers were dried (Na₂SO₄) and evaporated. The product was extracted from the residual solid with hot acetone (200 mL). Evaporation yielded 36 % of 3-methyl-pyrazin-2-ylamine **(2)** as a yellow solid.

### EXAMPLE 1: (3-Chloro-phenyl)-(8-methyl-2-phenyl-imidazo[1,2-a]pyrazin-3-yl)-amine (3)

### (General method A)

3-Methyl-pyrazin-2-ylamine **(2)** (109 mg, 1.0 mmol), benzaldehyde (106 mg, 1.0 mmol) and 3-chlorophenylisonitrile (138 mg, 1.0 mmol) were dissolved in a mixture of dry methanol (2.0 mL) and trimethyl orthoformate (2.0 mL) under argon. The mixture was stirred at 60°C for 3 hours, then cooled to rt. An analytically pure sample of **3** was obtained from the crude product using preparative HPLC.

### EXAMPLE 2: (3,4-Dichloro-phenyl)-(2-phenylimidazo[1,2-a]pyrazin-3-yl)-amine (29)

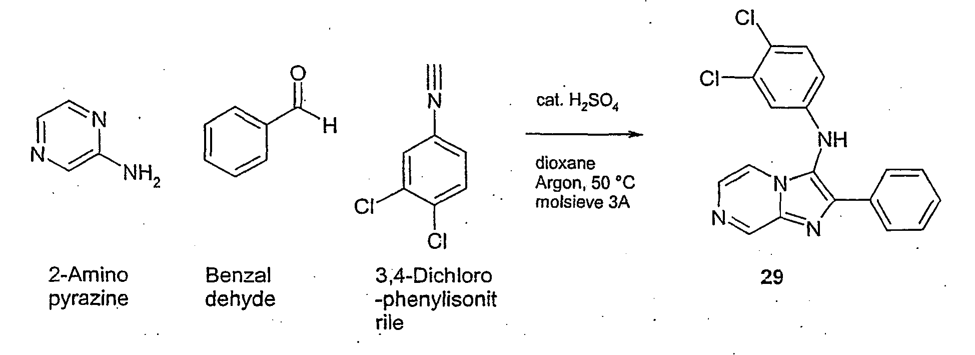

### (General method B)

2-Amino-pyrazine (95 mg, 1.0 mmol) and benzaldehyde (106 mg, 1.0 mmol) were dissolved in dry dioxane (2.0 mL) containing molecular sieves (3Å) under argon. After 5 minutes sulfuric acid (20µL) and 3,4-dichloro-phenylisonitrile (172 mg, 1.0 mmol) were added. The mixture was then stirred at 50°C for 3 hours, cooled to rt and filtered. The product **29** was obtained by crystallization from acetonitrile.

### EXAMPLE 3: N'8-Benzyl-N'3-(3-chloro-phenyl)-2-phenyl-imidazo[1,2-a]pyrazine-3,8-diamine (22)

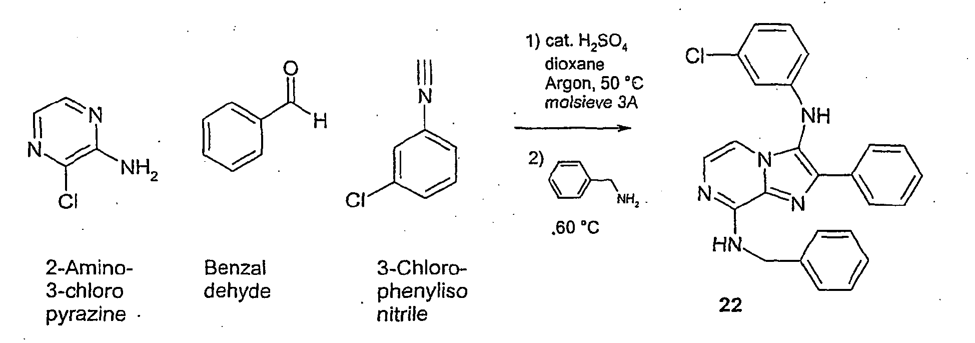

### (General method C for aminosubstituted derivatives)

2-Amino-3-chloro-pyrazine (130 mg, 1.0 mmol) and benzaldehyde (106 mug, 1.0 mmol) were dissolved in dry dioxane (3.0 mL) containing molecular sieves (3Å) under Argon. After 5 minutes sulfuric acid (20µL) and 3-chlorophenylisonitrile (138 mg, 1.0 mmol) were added. The mixture was then stirred at 50°C for 3 hours, cooled to rt and filtered.

Benzylamine (536 mg, 5.0 mmol) was added to the filtrate and the mixture was heated to 60°C for 20 h. After cooling to rt, filtration and evaporation to dryness, an analytically pure sample of **22** was obtained from the crude product using preparative HPLC.

### EXAMPLE 4: [2-(4-Amino-phenyl)-imidazol[1,2-a]pyrazin-3-yl]-(3-chloro-phenyl)-amine (10)

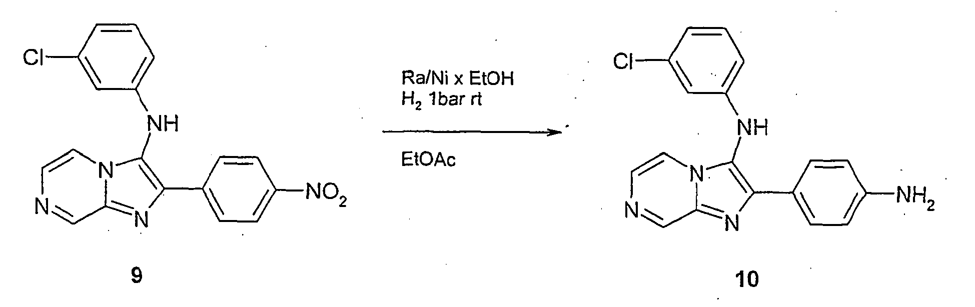

### (General method D for amino-derivatives via catalytic reduction of their nitro-precursors)

**9** (82 mg, 0.22 mmol) was dissolved in ethyl acetate (20 mL). 50 mg of Ra/Ni x EtOH were added and the mixture was hydrogenated (1 bar) at rt for 40 hours. The catalyst was filtered off, washed with ethyl acetate and the filtrate was evaporated to yield product **10** as a yellow solid. Pure **10** was obtained by crystallization from acetonitrile.

9 was prepared in analogy to General Method **A** using 4-nitrobenzaldehyde.

### EXAMPLE 5: 3-[3-(3-Chloro-phenylamino)imidazo[1,2-a]pyrazin-2-yl]-phenol (32)

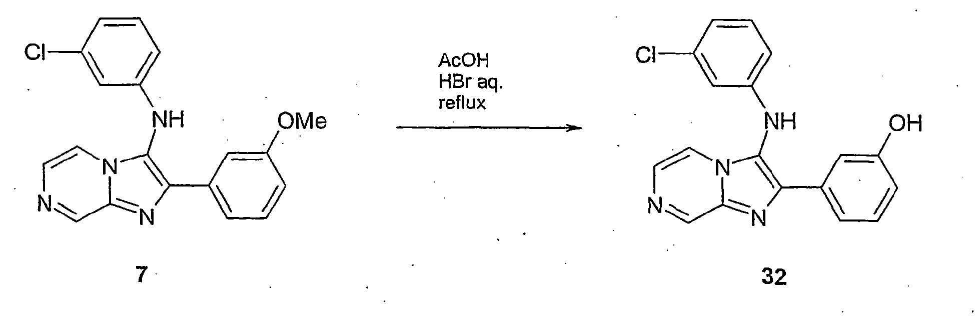

Synthesis of **32** via methyl ether cleavage of 7

7 was prepared in analogy to General Method **A** using 3-methoxybenzaldehyd. Pure 7 was obtained by crystallization from ethyl acetate / heptane.

7 (50 mg, 0.14 mmol) were refluxed in a mixture of glacial acetic acid (0.5 mL) and aqueous hydrobromic acid (5 mL) for 20 hours. After cooling to rt, it was extracted with dichloromethane (3 x 100 mL). The combined organic layers were was washed with water, saturated sodium bicarbonate solution and water, then dried (Na₂SO₄) and evaporated. **32** was obtained as a slight yellow solid.

### EXAMPLE 6: Additional compounds

The following compounds shown in TABLE 1, TABLE 2, TABLE 3 and TABLE 4 were prepared in accordance with the methods provided in Examples 1 to 4. Those of ordinary skill in the art of organic synthesis will recognize when starting materials or reaction conditions should be varied to obtain the desired compound.

The analytical data of the compounds is summarized in TABLE 5

**TABLE 1, compounds synthesized according to General Procedure A**

| **Structure** | **cpd** | **Pyrazine** | **Aldehyde** | **Isonitrile** |
|---|---|---|---|---|
| 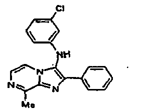 | 3 | 2 | Benzaldehyde | 3-Chlorophenylisonitrile |
| 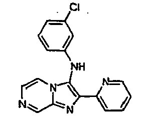 | **4** | 2-Amino-pyrazine | Pyridine-2-carbaldehyde | 3-Chlorophenylisonitrile |
| 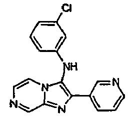 | **5** | 2-Amino-pyrazine | Pyridine-3-carbaldehyde | 3-Chlorophenylisonitrile |
| 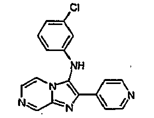 | **6** | 2-Amino-pyrazine | Pyridine-4-carbaldehyde | 3-Chlorophenylisonitrile |
| 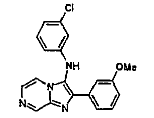 | **7** | 2-Amino-pyrazine | 3-Methoxybenzaldehyde | 3-Chlorophenylisonitrile |
| 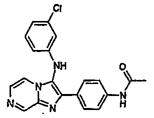 | **8** | 2-Amino-pyrazine | N-(4-Formylphenyl)-acetamide | 3-Chlorophenylisonitrile |
| 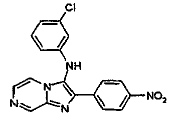 | **9** | 2-Amino-pyrazine | 4-Nitrobenzaldehyde | 3-Chlorophenylisonitrile |
| 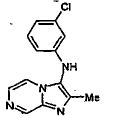 | **11** | 2-Amino-pyrazine | Acetaldehyde | 3-Chlorophenylisonitrile |
| 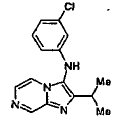 | **12** | 2-Amino-pyrazine | 2-Methylpropionaldehyde | 3-Chlorophenylisonitrile |
| 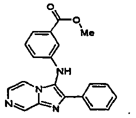 | **13** | 2-Amino-pyrazine | Benzaldehyde | 3-isocyano-benzoic acid methyl ester |
| 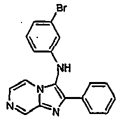 | **14** | 2-Amino-pyrazine | Benzaldehyde | 3-Bromophenylisonitrile |
| 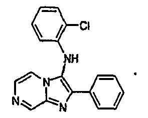 | **15** | 2-Amino-pyrazine | Benzaldehyde | 2-Chlorophenylisonitrile |
| 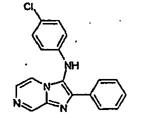 | **16** | 2-Amino-pyrazine | Benzaldehyde | 4-Chlorophenylisonitrile |
| 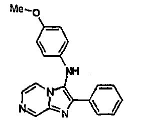 | **17** | 2-Amino-pyrazine | Benzaldehyde | 4-Methoxyphenylisonitrile |
| 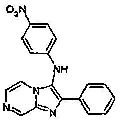 | **18** | 2-Amino-pyrazine | Benzaldehyde | 4-Nitrophenylisonitrile |
| 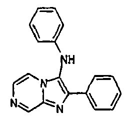 | **23** | 2-Amino-pyrazine | Benzaldehyde | Isocyano-benzene |
| 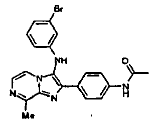 | **24** | **2** | N-(4-Formyl-phenyl)-acetamide | 3-Bromophenylisonitrile |
| 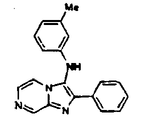 | **34** | 2-Amino-pyrazine | Benzaldehyde | 3-Methylphenylisonitrile |
| 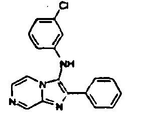 | **35** | 2-Amino-pyrazine | Benzaldehyde | 3-Chlorophenylisonitrile |
| 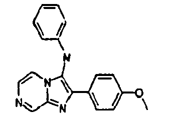 | **36** | 2-Amino-pyrazine | 4-Methoxybenzaldehyde | lsocyano-benzene |
| 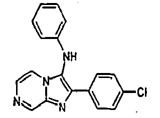 | **37** | 2-Amino-pyrazine | 4-Chlorobenzaldehyde | Isocyano-benzene |
| 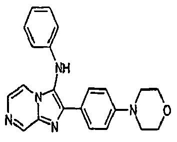 | **38** | 2-Amino-pyrazine | 4-Morpholinobenzaldehyde | Isocyano-benzene |
| 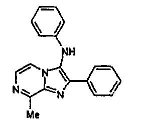 | **39** | **2** | Benzaldehyde | Isocyano-benzene |

**TABLE 2, compounds synthesized according to General Procedure B**

| **Structure** | **cpd** | **Pyrazine** | **Aldehyde** | **Isonitrile** |
|---|---|---|---|---|
| 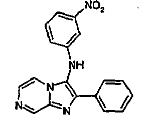 | **25** | 2-Amino-pyrazine | Benzaldehyde | 3-Nitrophenylisonitrile |
| 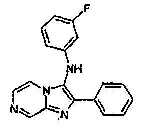 | **26** | 2-Amino-pyrazine | Benzaldehyde | 3-Fluorophenylisonitrile |
| 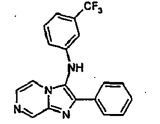 | **27** | 2-Amino-pyrazine | Benzaldehyde | 3-Trifluoromethylphenylisonitrile |
| 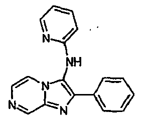 | **28** | 2-Amino-pyrazine | Benzaldehyde | 2-lsocyano-pyridine |
| 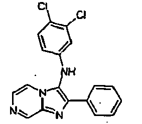 | **29** | 2-Amino-pyrazine | Benzaldehyde | 3,4-Di-chloro-3,4-Di-chlorophenylisonitrile |
| 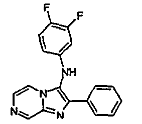 | **30** | 2-Amino-pyrazine | Benzaldehyde | 3,4-Di-fluorophenylisonitrile |
| 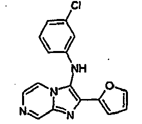 | **31** | 2-Amino-pyrazine | Furfualdehyde | 3-Chlorophenylisonitrile |
| 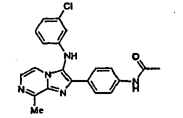 | **33** | **2** | N-(4-Formylphenyl)-acetamide | 3-Chlorophenylisonitrile |
| 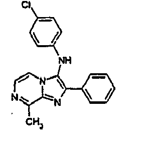 | **41** | **2** | Benzaldehyde | 4-Chlorophenylisonitrile |
| 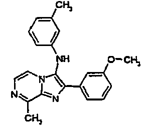 | **42** | **2** | 3-Methoxybenzaldehyde | 3-Methylphenylisonitrile |
| 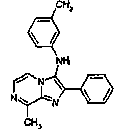 | **43** | **2** | Benzaldehyde | 3-Methylphenylisonitrile |
| 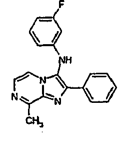 | **44** | **2** | Benzaldehyde | 3-Fluorophenylisonitrile |
| 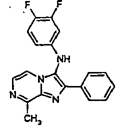 | **45** | **2** | Benzaldehyde | 3,4-Di-fluorophenylisonitrile |
| 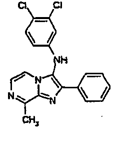 | **46** | **2** | Benzaldehyde | 3,4-Di-chlorophenylisonitrile |
| 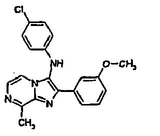 | **47** | **2** | 3-Methoxybenzaldehyde | 4-Chlorophenylisonitrile |
| 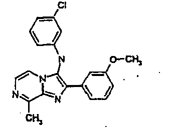 | **48** | **2** | 3-Methoxybenzaldehyde | 3-Chlorophenylisonitrile |

**TABLE 3, compounds synthesized according to General Procedure C**

| **Structure** | **cpd** | **Pyrazine** | **Aldehyde** | **Isonitrile** | **Amine** |
|---|---|---|---|---|---|
| 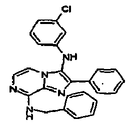 | **22** | 2-Amino-3-chloro-pyrazine | Benzaldehyde | 3-Chlorophenylisonitrile | Benzylamine |
| 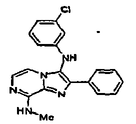 | **20** | 2-Amino-3- | Benzaldehyde chioro-pyrazine sonitrite | 3-Chlorophenylisonitrile | Methylamine |
| 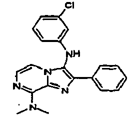 | **21** | 2-Amino-3-chloro-pyrazine | Benzaldehyde | 3-Chlorophenylisonitrile | Dimethylamine |

**TABLE 4, compounds synthesized according to General Procedure D**

| **Structure** | **cpd** | **Synthesis** |
|---|---|---|
| 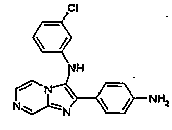 | **10** | from 9 |
| 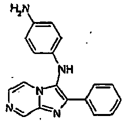 | **19** | from 18 |
| 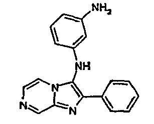 | **40** | from 25 |

**TABLE 5, analytical data**

| **Cpd** | **Name** | **Rt (HPLC) min.** | **Rt (LC-MS) min.** | **m/z [M+H]+** | **¹H NMR (300 MHz, DMSO) characteristic signals [ppm]** |
|---|---|---|---|---|---|
| **3** | (3-Chloro-phenyl)-(8-methyl-2-phenylimidazo[1,2-a]pyrazin-3-yl)-amine | 2.70 | 1.97 | 335 | 8.76, br, 1H; 7.82, d, 4.7 Hz, 1H; 2.87, s, 3H |
| **4** | (3-Chloro-phenyl)-(2-pyridin-2-yl-imidazo[1,2-a]pyrazin-3-yl)-amine | 2.02 | 1.72 | 322 | 9.14, d, 1.5 Hz; 8.75, s, 1H; 8.61, m, 1H |
| **5** | 3-Chloro-phenyl)-(2-pyridin-3-yl-imidazo[1,2-a]pvrazin-3-yl)-amine | 1.69 | 1.48 | 322 | 9.19, m, 2H; 8.73, s, 1H; 8.56, m, 1H |
| **6** | (3-Chloro-phenyl)-(2-pyridin-4-yl-imidazo[1,2-alpyrazin-3-yl)-amine | 1.79 | 1.25 | 322 | 9.17, d, 1.4 Hz, 1H;8.85, br, 1H;6.80, m, 2H |
| **7** | (3-Chloro-phenyl)-[2-(3-methoxy-phenyl)-imidazo[1,2-a]pyrazin-3-yl]-amine | 2.68 | 1.92 | 351 | 9.13, d, 1.5 Hz, 1H; 8.67, s, 1H; 3.73, s, 3H |
| **8** | N-{4-[3-(3-Chlorophenylamino)-imidazo[1,2-a]pyrazin-2-yl]-phenyl}-acetamide | 2.24 | 1.63 | 378 | 10.1, br, 1H; 9.24, d, 1.4 Hz, 1H; 8.76, br, 1H; 2.06, s, 3H |
| **9** | (3-Chloro-phenyl)-[2-(4-nitro-phenyl)-imidazo[1,2-a]pyrazin-3-yl]-amine | 2.90 | 1.99 | 366 | 9.19, br, 1H; 8.82, br, 1H; 6.67, br, 1H |
| **10** | [2-(4-Amino-phenyl)-imidazo[1,2-a]pyrazin-3-yl]-(3-chloro-phenyl)-amine | 1.89 | 0.85 | 336 | 9.00, br, 1H; 8.51, br, 1H; 8.00, m, br, 1H |
| **11** | (3-Chloro-phenyl)-(2-methyl-imidazo[1,2-a]pyrazin-3-yl)-amine | 2.04 | 1.51 | 259 | 9.20, d, 1.1 Hz, 1H; 8.60, br, 1H; 2.36, s, 3H |
| **12** | (3-Chlorophenyl)-(2-isopropyl-imidazo[1,2-alpyrazin-3-yl)-amine | 2.38 | 1.77 | 287 | 9.33, d, 0.8 Hz, 1H; 8.63, br, 1H; 1.34, d, 6.9 Hz, 6H |
| **13** | 3-(2-Phenylimidazo[1,2-a]pyrazin-3-ylamino)-benzoic acid methyl ester | 2.46 | 1.74 | 345 | 9.18, br, 1H; 8.76, br, 1H; 3.82, s, 3H |
| **14** | (3-Bromophenyl)-(2-phenylimidazo[1,2-alpyrazin-3-yl)-amine | 2.81 | 1.94 | 365 | 9.22, d, 0.9 Hz; 8.74, br, 1H; 6.48, m, 1H |
| **15** | (2-Chlorophenyl)-(2-phenylimidazo[1,2-a]pyrazin-3-yl)-amine | 2.79 | 1.94 | 321 | 9.14, d, 1.5 Hz, 1H; 8.15, s, 1H; 6.08, m, 1H |
| **16** | (4-Chlorophenyl)-(2-phenylimidazo[1,2-a]pyrazin-3-yl)-amine | 2.80 | 1.93 | 321 | 9.11, d, 1.4 Hz, 1H; 8.57, s, 1H; 6.54, m, 1H |
| **17** | (4-Methoxyphenyl)-(2-phenylimidazo[1,2-a]pyrazin-3-yl)-amine | 2.41 | 1.70 | 317 | 9.09, d, 1.5 Hz, 1H; 5.76, s, 1H; 3.64, s, 3H |
| **18** | (4-Nitrophenyl)-(2-phenylimidazo[1,2-a]pyrazin-3-yl)-amine | 2.34 | 1.72 | 332 | 9.54, br, 1H; 9.19, br, 1H; 6.71, m, 2H |
| **19** | N-(2-Phenylimidazo[1,2-a]pyrazin-3-yl)-benzene-1,4-diamine | | 0.79 | 302 | |
| **20** | N'3-(3-Chloro-phenyl)-N'8-methyl-2-phenylimidazo[1,2-a]pyrazine-3,8-diamine | 2.49 | 1.64 | 350 | 8.44, br, 1H;7.90, m, 2H;2.92, d, 4.8 Hz, 3H |
| **21** | N'3-(3-Chloro-phenyl)-N'8,N'8-dimethyl-2-phenylimidazo[1,2--a]pyrazine-3,8-diamine | | 1.66 | 364 | |
| **22** | N'8-Benzyl-N'3-(3-chlorophenyl)-2-phenyl-imidazo[1,2-a]pyrazine-3,8-diamine x HCl | 2.93 | 2.22 | 426 | 8.95, br, 1H;7.99, m, 2H;4.95, m, 2H |
| **23** | Phenyl-(2-phenyl-imidazo[1,2-a]pyrazin-3-yl)-amine | 2.36 | 1.75 | 287 | 9.11, d, 1.4Hz, 1H; 8.41, br, 1H; 6.52, m, 2H |
| **24** | N-{4-[3-(3-Bromo-phenylamino)-8-methyl-imidazo[1,2-a]pyrazin-2-yl]-phenyl}-acetamide | 2.08 | 1.68 | 436 | 10.0, br, 1H; 8.59, br, 1H; 2.05, s, 3H |
| **25** | (3-Nitrophenyl)-(2-phenyl-imidazo[1,2-a]pyrazin-3-yl)-amine | 2.35 1.75 | | 332 | 9.15, d, 1.5 Hz, 1H; 9.03, s, 1H; 8.85, m, 1H; |
| **26** | (3-Fluorophenyl)-(2-phenyl-imidazo[1,2-a]pyrazin-3-yl)-amine | 2.36 | 1.81 | 305 | 9.23, d, 1.3 Hz, 1H; 8.76, br, 1H; 6.31, m, 2H |
| **27** | (2-Phenylimidazo[1,2-a]pyrazin-3-yl)-(3-trifluoromethy)-phenyl)-amine | 2.65 | 1.97 | 355 | 9.18, d, 1.5 Hz, 1H;8.88, s, 1H;6.71, m, 1H |
| 28 | (2-Phenylimidazo[1,2-a]pyrazin-3-yl)-pyridin-2-yl-amine | 1.41 | 1.29 | 288 | 9.11, d, 1.4Hz, 1H;8.06, m, 1H;8.80, m, 2H |
| **29** | (3,4-Dichlorophenyl)-(2-phenyl-imidazo[1,2-a]pyrazin-3-yl)-amine | 2.76 | 2.03 | 355 | 9.15, d, 1.4 Hz, 1H; 8.81, s, 1H; 6.50, m, 1H |
| **30** | (3,4-Difluorophenyl)-(2-phenyl-imidazo[1,2-a]pyrazin-3-yl)-amine | 2.43 | 1.85 | 323 | 9.11, d, 1.5 Hz, 1H;8.58, s, 1H;6.58, m, 1H |
| **31** | (3-Chlorophenyl)-(2-furan-2-yl-imidazo[1,2-a]pyrazin-3-yl)-amine | 2.31 | 1.74 | 311 | 9.08, d, 1.5 Hz, 1H;8.58, s, 1H;6.43, m, 1H |
| **32** | 3-[3-(3-Chlorophenylamino) imidazo[1,2-a]pyrazin-2-yl]-Phenol | 2.22 | 1.70 | 337 | 9.32, br, 1H;8.86, br, 1H;6.50, m, 1H |
| **33** | N-{4-[3-(3-Chloro-phenylamino)-8-methyl-imidazo[1,2-a]pyrazin-2-yl]-phenyl}-acetamide | | 1.69 | 392 | |
| **34** | (2-Phenyl-imidazo[1,2-a]pyrazin-3-yl)-m-tolylamine | | 1.89 | 301 | 9.11, d, 1.5 Hz, 1H; 8.33, s, 1H; 2.16, s, 3H |
| **35** | (3-Chloro-phenyl)-(2-phenyl-imidazo[1,2-a]pyrazin-3-yl)-amine | | 1.92 | 321 | 9.12, d, 1.4 Hz, 9H; 8.67, br, 1H; 6.42, m, 2H |
| **36** | [2-(4-Methoxy-phenyl)-imidazo[1,2-a]pyrazin-3-yl]-phenyl-amine | | 1.76 | 317 | 9.06, d, 1.5 Hz; 8.34, s, 1H; 3.77, s, 3H |
| **37** | [2-(4-Chloro-phenyl)-imidazo[1,2-a]pyrazin-3-yl]-phenyl-amine | | 1.96 | 321 | 9.11, d, 1.4Hz, 1H; 8.42, s, 1H; 6.51, d, 7.6 Hz, 2H |
| **38** | [2-(4-Morpholin-4-yl-phenyl)-imidazo[1,2-a]pyrazin-3-yl]-phenylamine | | 1.69 | 372 | 9.03, d, 1.5Hz, 1H; 3.72, m, 2H; 3.15, m, 2H |
| **39** | (8-Methyl-2-phenylimidazo[1,2-a]pyrazin-3-yl)-phenyl-amine | | 1.80 | 301 | 8.31, br, 1H; 8.00, m, 2H; 2.74, s, 3H |
| **40** | N-(2-Phenylimidazo[1,2-a]pyrazin-3-yl)-benzene-1,3-diamine | | 1.24 | 302 | |
| **41** | (4-Chloro-phenyl)-(8-methyl-2-phenylimidazo[1,2-a]pyrazin-3-yl)-amine | 2.51 | 1.98 | 335 | 8.55, s, 3H; 8.05, m, 2H; 2.82, s, 3H |
| **42** | [2-(3-Methoxy-phenyl)-8-methyl-imidazo[1,2-a]pyrazin-3-yl]-m-tolylamine | 2.44 | 1.92 | 345 | 8.30, s, 1H; 3.70, s, 3H; 2.15, s, 3H |
| **43** | (8-Methyl-2-phenylimidazo[1,2-a]pyrazin-3-yl)-m-tolyl-amine | 2.41 | 1.92 | 315 | 8.30, s, 1H; 2.82, s, 3H; 2.15, s, 3H |
| **44** | (3-Fluoro-phenyl)-(8-methyl-2-phenylimidazo[1,2-a]pyrazin-3-yl)-amine | 2.36 | 1.88 | 319 | 8.65, br, 1H; 6.30, m, 2H; 2.82, s, 3H |
| **45** | (3,4-Difluoro-phenyl)-(8-methyl-2-phenylimidazo[1,2-a]pyrazin-3-yl)-amine | 2.41 | 1.92 | 337 | 8.57, s, 3H; 6.28, m, 1H; 2.81, s, 3H |
| **46** | (3,4-Dichloro-phenyl)-(8-methyl-2-phenylimidazo[1,2-a]pyrazin-3-yl)-amine | 2.69 | 2.12 | 369 | 8.88, br, 1H; 6.50, m, 1H; 2.87, s, 3H |
| **47** | (4-Chloro-phenyl)-[2-(3-methoxy-phenyl)-8-methyl-imidazo[1,2-a]pyrazin-3-yl]-amine | 2.52 | 2.00 | 365 | 8.55, br, 1H; 3.71, s, 3H; 2.81, s, 3H |
| **48** | (3-Chloro-phenyl)-[2-(3-methoxy-phenyl)-8-methyl-imidazo[1,2-a]pyrazin-3-yl]-amine | 2.51 | 1.98 | 365 | 8.65, br, 1H; 3.72, s, 3H; 2.82, s, 3H |

### EXAMPLE 7: Assay for EPHB4 kinase activity

### Kinase assay protocol

The kinase inhibition activity of the compounds was measured in an *in vitro* kinase assay.

Briefly, in a final reaction volume of 25 µL, human EphB4 (N-terminal His6-tagged, recombinant, amino acids 561-end, expressed by baculovirus in Sf21 insect cells; 5-10 mU) was incubated with 8 mM MOPS pH 7.0, 0.2 mM EDTA, 10 mM MnCl₂, 0.1 mg/mL poly(Glu, Tyr) 4:1, 10 mM MgAcetate and [γ-³³p-ATP] (specific activity approx. 500 cpm/pmol, concentration as required). The reaction was initiated by the addition of the MgATP mix. After incubation for 40 min at rt, the reaction was stopped by the addition of 5 µl of a 3% phosphoric acid solution. 10 µL of the reaction was then spotted onto a Filtermat A and washed three times for 5 min in 75 mM phosphoric acid and once in methanol prior to drying and scintillation counting.

### EXAMPLE 8: Test results

All compounds described in Example 1 to 6 were tested in the assay for EPHB4 activity described in Example 7 and found to exhibit an IC₅₀ of 6 µM or less. Certain compounds disclosed in Example 1 to 6 exhibited an IC₅₀ of 500 nM or less in this assay. A subset of those compounds even exhibited an IC₅₀ of 150 nM or less.

While certain embodiments have been shown and described, numerous modifications and substitutions may be made without departing from the scope of the invention. Therefore, present invention has been described by way of examples and they have to be understood in an illustrative sense only and are not to be interpreted as limiting this invention in any manner.

### EXAMPLE 9: Cellular autophosphorylation assay (ELISA)

CHO (chinese hamster ovary) cells were stably transfected with myc-tagged full length human EphB4. A cell line expressing EphB4 of which the phosphorylation was inducible with ephrinB2/Fc was identified and called CHO-EphB4.

CHO-EphB4 cells were seeded into a 24-well plate and cultured overnight in HAM-F12/10% FCS/600 µg/ml hygromycine B. The cells were washed once with PBS and dilutions of compounds in Ham-F12 medium were added (in triplicates). Cells were incubated at 37°C for 15 min-utes. Ligand ephrinB2/Fc (R&D Systems) was added (15 mM final) and cells were further incubated at 37°C for 45 minutes. Cells were lysed in 120 µl/well of lysis buffer (20 mM Tris pH8, 150 mM NaCl, 10% glycerol, 1% TritonX-100, 1 mM Na₃VO₄, 1 mM EDTA pH8, 1 mM EGTA pH8, 1x protease inhibitor cocktail Sigma). ELISA 96-well plates (Greiner, Polysorb) were coated with 100 µl/well anti-c-myc antibody (Sigma) overnight at 4°C, blocked with 300 µl/well 5% milk in TBS/0.005% Tween (TBST) for 1 to 3 hours at room temperature, and incubated with 110 µl/well cell lysate overnight at 4°C. ELISA plates were washed twice with TBST and incubated with 100 µl/well anti-P-Tyr-HRP antibody (Upstate) diluted 1:10000 in 5% milk in TBST for 1.5 hour at RT and developed with 100 µl/well BM BluePOD substrate (Roche). Absorbance was measured at 450 nm.

### Results:

Compounds measured in the cellular autophosphorylation assay showed IC50s in the range of 10 µM to higher concentrations; a subset of compounds showed IC50s in the range of 1-10 µM.

### EXAMPLE 10: Specificity profiling: yeast growth assay

The yeast growth assay was performed as described in "Method for the identification and/or validation of receptor tyrosine kinase inhibitors" PCT/CH03/00694 (2004/10/24), section "Modes for Carrying out the Invention, Experiment 1 and Experiment 2.

Briefly, yeast cells carrying plasmids with the RTK of interest were grown in glucose containing medium (repressing expression). The cultures were diluted into galactose containing medium, which induces the expression of the kinase. Expression of the kinase inhibits growth of the yeast cells, which allows for positive selection of kinase inhibitors restoring growth of cells. Compounds were titrated at 7 concentrations between 0.2 and 100 µM.

### Results:

The results are summarized in Table 6. Specificity profiling of compounds, using the yeast growth assay method described above. "-" indicates no growth restoration i.e. no activity against the particular kinase, "+", "++", and "+++" indicate activity detected by restoration of growth, classified into three categories of potency according to the maximal level of growth restoration reached and the concentration at which the maximal growth restoration is reached.

**TABLE 6: Specificity profiling of compounds**

| **cpd n°** | **EphB4 34** | **EphB2** | **EphA2** | **RET** | **EGFR(L858R)** | **ErbB2** |
|---|---|---|---|---|---|---|
| **34** | + - | - | - | - | - | - |
| **35** | + | - | - | + | + | - |
| **39** | +++ | +++ | + | + | + | - |
| **3** | ++ | ++ | - | - | + | - |
| **7** | + | - | - | + - | + | - |
| **14** | - | - | - | - | - | - |
| **16** | + | - | - | + | - | - |
| **43** | +++ | ++ | - | - | - | - |
| **44** | +++ | +++ | - | - | + | - |
| **45** | +++ | ++ | - | - | - | - |
| **23** | ++ | ++ | + | + | + | - |
| **42** | ++ | +++ | + | - | (+) | ND |
| **48** | ++ | +++ | + | - | (+) | ND |
| **10** | +++ | ++ | ++ | - | (+) | ND |
| **26** | ++ | + | - | - | (+) | ND |
| **30** | ++ | + | - | - | (+) | ND |
| **33** | +++ | +++ | + | - | (+) | ND |
| **38** | ++ | ++ | + | - | (+) | ND |
| **12** | + | + | + | - | (+) | ND |
| **8** | ++ | ++ | + | - | (+) | ND |
| **20** | - | - | - | - | (-) | ND |

| | | | | | | |
|---|---|---|---|---|---|---|
| (+) in a different yeast background ND not determined | | | | | | |

| **cpd n°** | **InsR** | **IGF-1R** | **PDGFRa** | **PDGFRb** | **KIT** | **VEGFR1** | **VEGFR2** |
|---|---|---|---|---|---|---|---|
| **34** | - | - | - | - | - | - | - |
| **35** | - | - | - | - | - | - | - |
| **39** | - | - | - | - | - | - | - |
| **3** | - | - | - | - | - | - | - |
| **7** | - | - | - | - | - | - | - |
| **14** | - | - | - | - | - | - | - |
| **16** | - | - | - | - | - | - | - |
| **43** | - | - | - | - | - | - | - |
| **44** | - | - | - | - | - | - | - |
| **45** | - | - | - | - | - | - | - |
| **23** | - | - | - | - | - | - | - |
| **42** | - | - | ND | ND | ND | - | - |
| **48** | - | - | ND | ND | ND | - | - |
| **10** | - | - | ND | ND | ND | - | - |
| **26** | - | - | ND | ND | ND | - | - |
| **30** | - | - | ND | ND | ND | - | - |
| **33** | - | - | ND | ND | ND | - | - |
| **38** | + | - | ND | ND | ND | + | - |
| **12** | - | - | ND | ND | ND | - | - |
| **8** | - | - | ND | ND | ND | - | - |
| **20** | - | - | ND | ND | ND | - | - |

| **cpd n°** | **FLT3** | **TEK** | **CSF-1R** | **FGFR-1** | **FGFR-2** | **FGFR-3** | **FGFR-4** |
|---|---|---|---|---|---|---|---|
| **34** | - | - | - | - | - | - | - |
| **35** | - | - | - | - | - | - | - |
| **39** | - | - | - | - | - | - | - |
| **3** | - | - | - | - | - | - | - |
| **7** | - | - | - | - | - | - | - |
| **14** | - | - | - | - | - | - | - |
| **16** | - | - | - | - | - | - | - |
| **43** | - | - | - | - | - | - | - |
| **44** | - | - | - | - | - | - | - |
| **45** | - | - | | - | - | - | - |
| **23** | - | - | - | - | - | - | - |
| **42** | ND | - | ND | ND | ND | ND | ND |
| **48** | ND | - | ND | ND | ND | ND | ND |
| **10** | ND | - | ND | ND | ND | ND | ND |
| **26** | ND | - | ND | ND | ND | ND | ND |
| **30** | ND | - | ND | ND | ND | ND | ND |
| **33** | ND | - | ND | ND | ND | ND | ND |
| **38** | ND | - | ND | ND | ND | ND | ND |
| **12** | ND | - | ND | ND | ND | ND | ND |
| **8** | ND | - | ND | ND | ND | ND | ND |
| **20** | ND | - | ND | ND | ND | ND | ND |

Enlarged structural formulas of the compounds of Tables 1 to 4

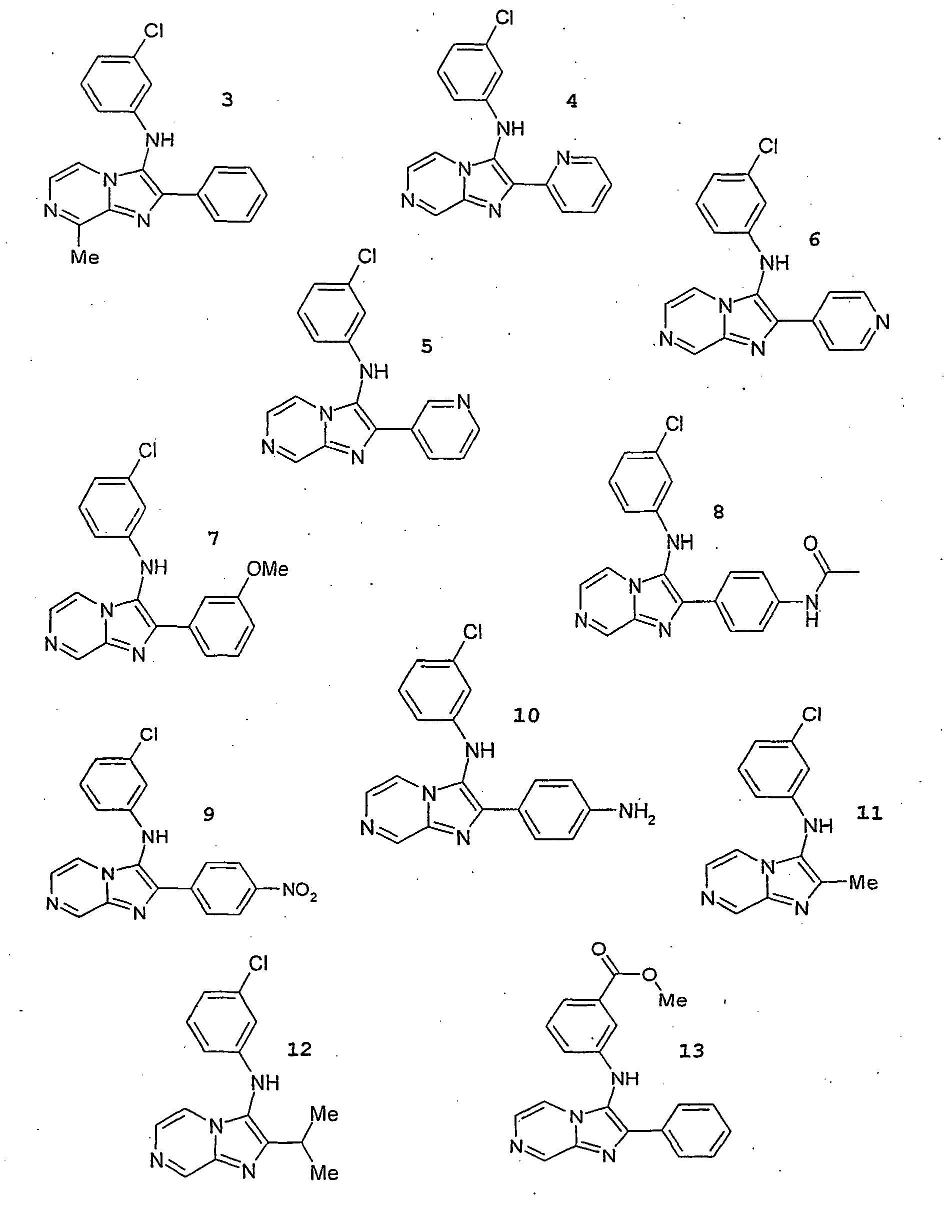

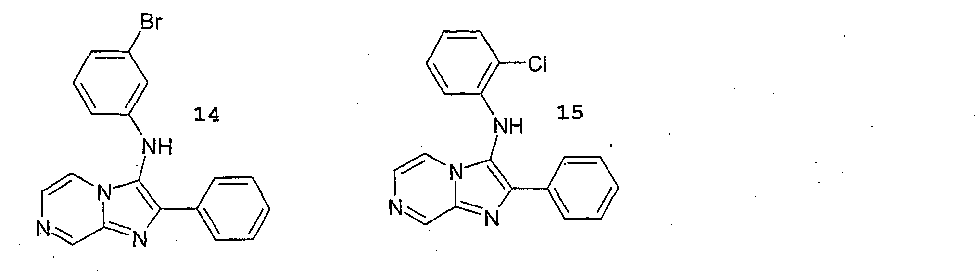

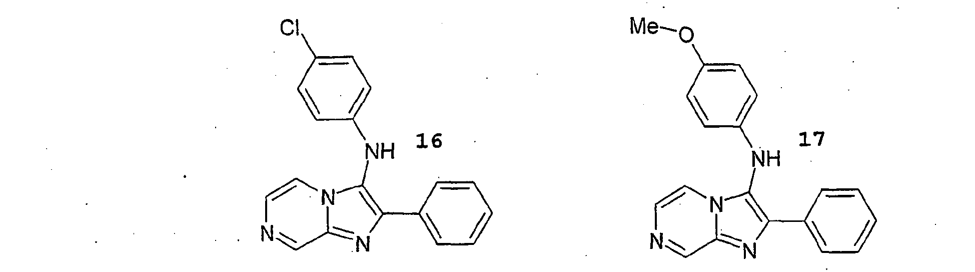

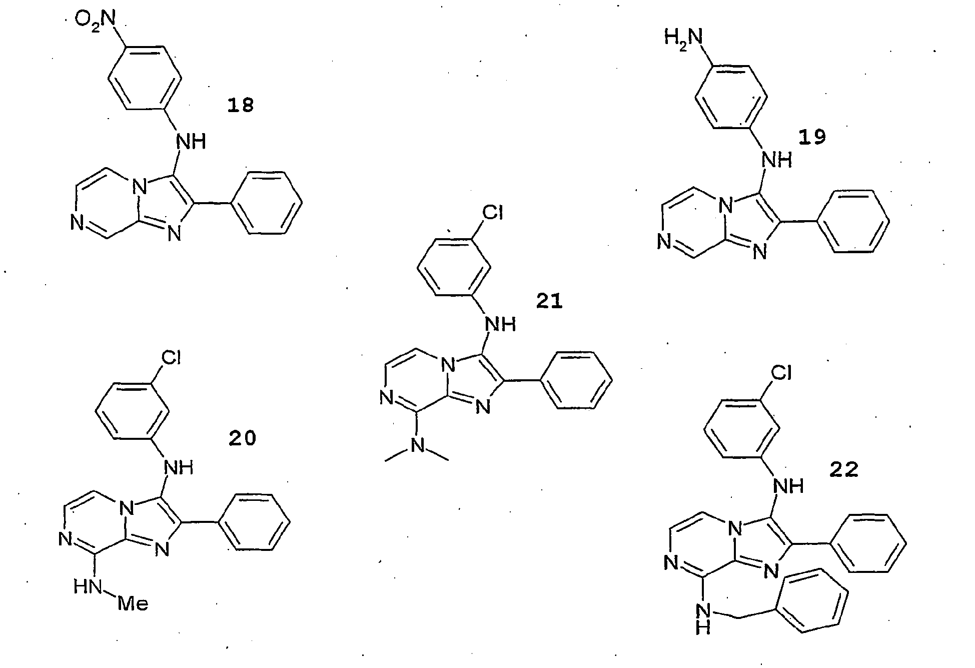

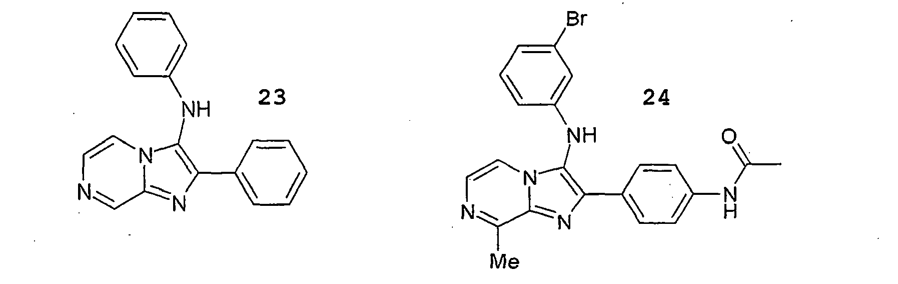

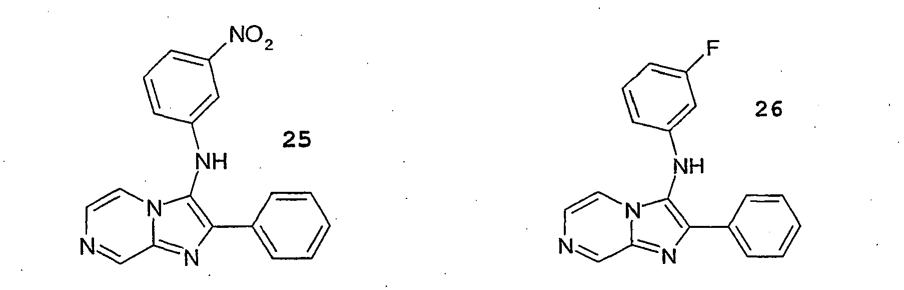

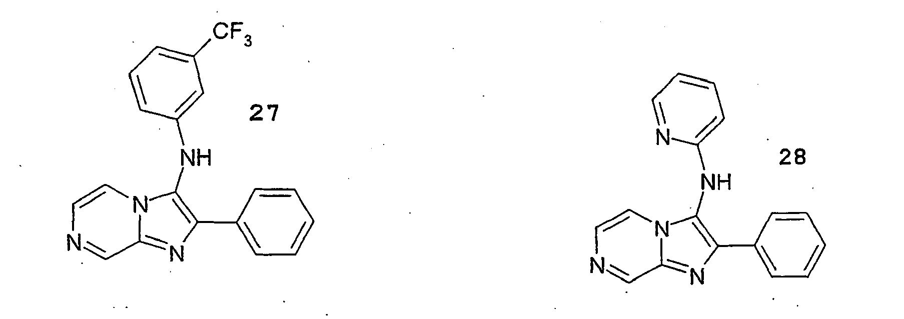

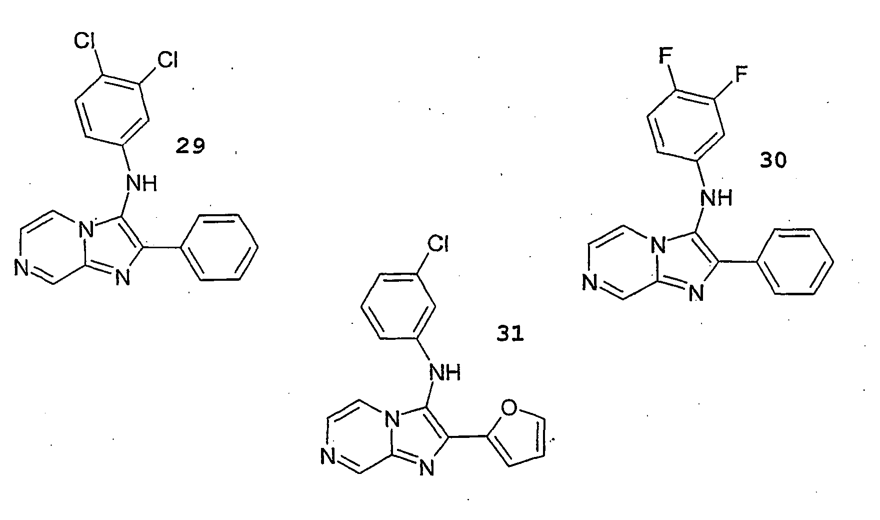

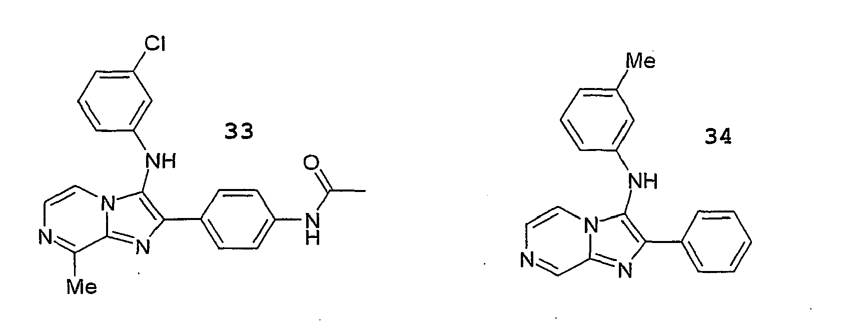

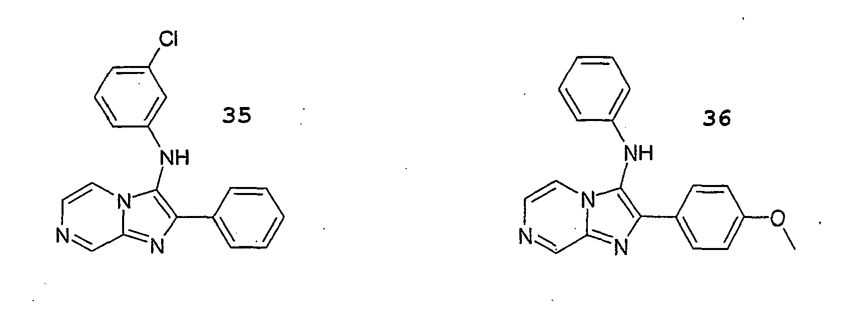

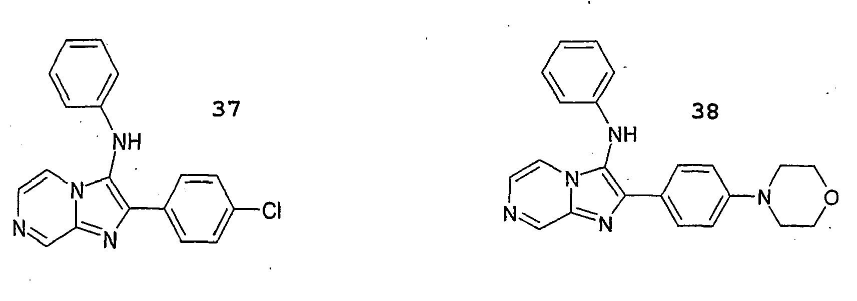

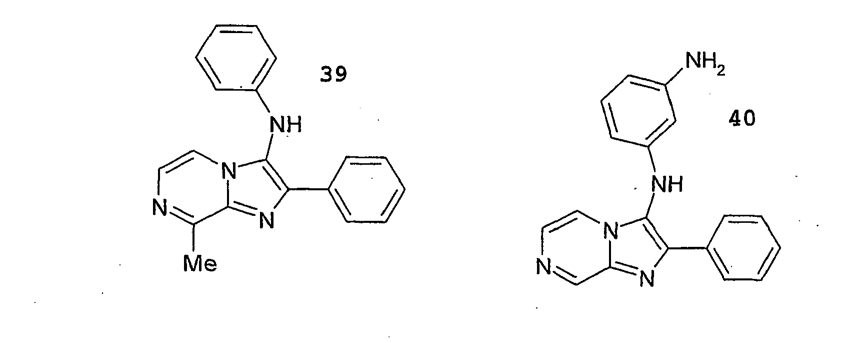

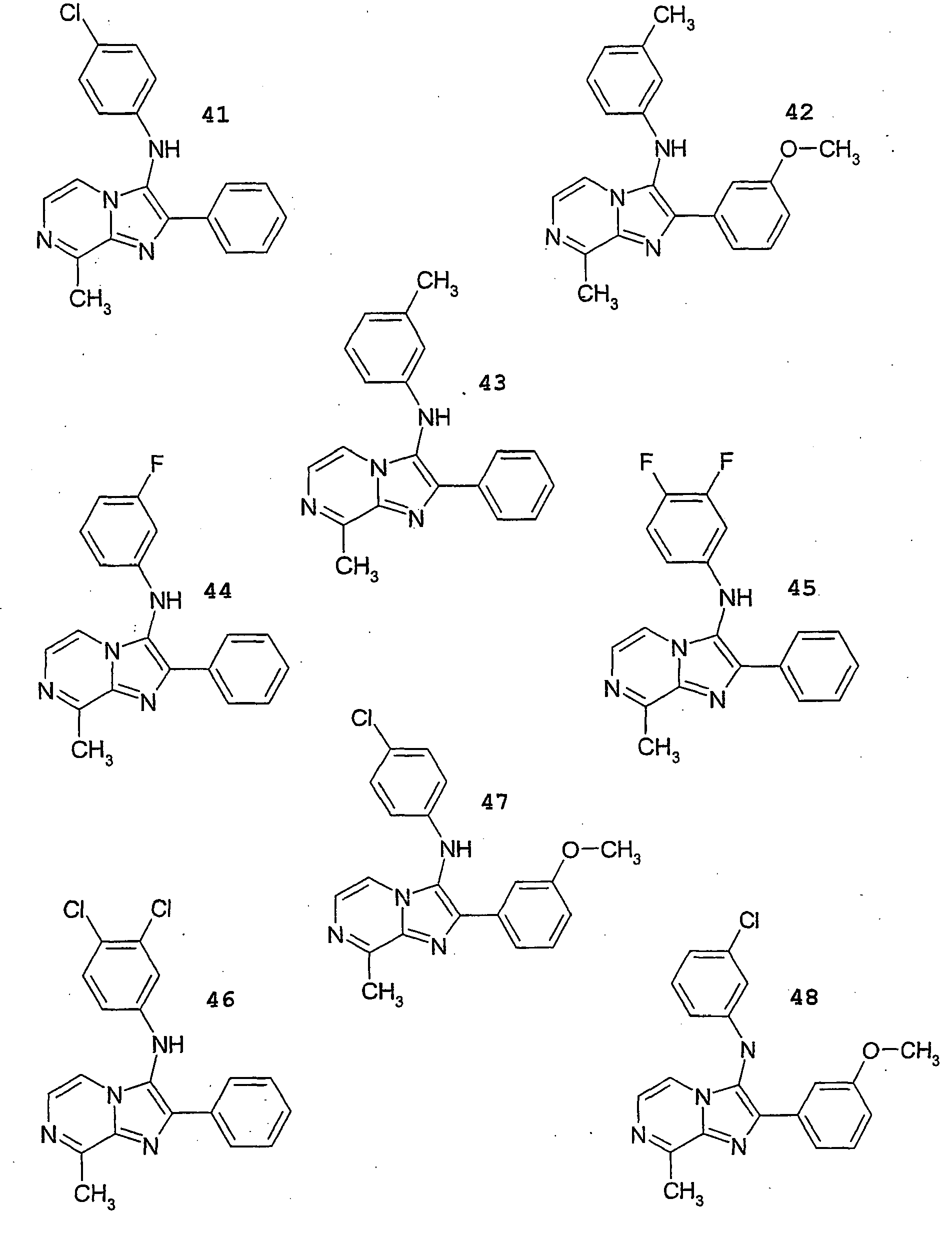

### References:

Berclaz, G., E. Karamitopoulou, et al. (2003). "Activation of the receptor protein tyrosine kinase EphB4 in endometrial hyperplasia and endometrial carcinoma." Ann Oncol 14(2): 220-6.
Brantley-Sieders, D., M. Parker, et al. (2004). "Eph receptor tyrosine kinases in tumour and tumor microenvironment." Curr Pharm Des 10(27): 3431-42.
Brantley-Sieders, D. M. and J. Chen (2004). "Eph receptor tyrosine kinases in angiogenesis: from development to disease." Angiogenesis 7(1): 17-28.
Cheng, N., D. M. Brantley, et al. (2002). "The ephrins and Eph receptors in angiogenesis." Cytokine Growth Factor Rev 13(1): 75-85.
Davy, A. and P. Soriano (2005). "Ephrin signaling in vivo: look both ways." Dev Dyn 232(1): 1-10.
Dodelet, V. C. and E. B. Pasquale (2000). "Eph receptors and ephrin ligands: embryogenesis to tumorigenesis." Oncogene 19(49): 5614-9.
Liu, W., S. A. Ahmad, et al. (2002). "Coexpression of ephrin-Bs and their receptors in colon carcinoma." Cancer 94(4): 934-9.
Klagsbrun, M. and And A. Eichmann (2005). "A role for axon guidance receptors and ligands in blood vessel development and tumor angiogenesis". Cytokine & Growth Factor Rev 16: 535-548.
Lugli, A., H. Spichtin et al. (2005). "EphB2 expression across 138 human tumor types in a tissue microarray: high levels of expression in gastrointestinal cancers." Clin Cancer res 11(18): 6450-6458.
Munarini, N., R. Jager, et al. (2002). "Altered mammary epithelial development, pattern formation and involution in transgenic mice expressing the EphB4 receptor tyrosine kinase." J Cell Sci 115(Pt 1): 25-37.
Pasquale, E. B. (2005). "Eph receptor signalling casts a wide net on cell behaviour." Nat Rev Mol Cell Biol 6(6): 462-75.
Stacker, S.A., R.A. Hughes, et al. (2004). "Molecular targeting of lymphatics for therapy". Curr Pharm Des. 10(1): 65-74.
Tammela, T., T.V. Petrova, et al. (2005). "Molecular lymphangiogenesis: new players". Trends Cell Biol 15(8): 434-440.
Surawska, H., P. C. Ma, et al. (2004). "The role of ephrins and Eph receptors in cancer." Cytokine Growth Factor Rev 15(6): 419-33.
Wang, Z., K. Cohen, et al. (2004). "Ephrin receptor, EphB4, regulates ES cell differentiation of primitive mammalian hemangioblasts, blood, cardiomyocytes, and blood vessels." Blood 103(1): 100-9.
Wang, Z., N. Miura, et al. (2002). "Receptor tyrosine kinase, EphB4 (HTK), accelerates differentiation of select human hematopoietic cells." Blood 99(8): 2740-7.
Xia, G., S. R. Kumar, et al. (2005). "EphB4 receptor tyrosine kinase is expressed in bladder cancer and provides signals for cell survival." Oncogene: 1-12.

## Claims

1. Use of a kinase inhibitor of formula I 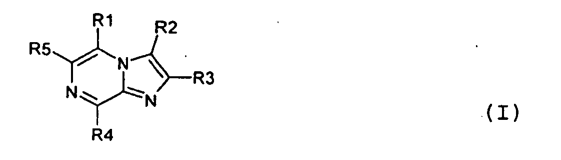 wherein
R1 and R5 are independently selected from hydrogen, halogen, cyano, nitro, CF₃, optionally substituted linear or branched C₁-C₆ alkyl, optionally substituted C₁-C₆ alkenyl, optionally substituted C₁-C₆ alkinyl, optionally substituted C₃-Cg cycloalkyl, alkoxy, NR₆R₇, OR₆, SR₆, (CH₂)ₙCHR₆R₇, wherein n is 0, 1, 2, 3, 4 and R₆ and R₇ are independently selected from hydrogen, optionally substituted C₃₋-C₈ cycloalkyl, optionally substituted aryl, optionally substituted 5- or 6-membered heterocycle, (CH₂)ₙC(O)OR₈, (CH₂)ₙR₉, wherein n is as defined above, R₈ is hydrogen, optionally substituted C₁-C₆ alkyl, optionally substituted C₃-C₈ cycloalkyl, optionally substituted aryl and R₉ is selected from hydrogen, C₁-C₆ alkoxy, optionally substituted C₃-C₈ cycloalkyl, optionally substituted aryl, halogen, hydroxyl, NO₂, NH₂, SO₂NH₂, cyano, and wherein all optional substituents are selected from the group of methyl, methoxy, halogen, OH, NO₂ NH₂, -C(O)OMe, cyano;
R₂ and R₄ are independently selected from hydrogen, optionally substituted linear or branched C₁-C₆ alkyl, optionally substituted C₁-C₆ alkenyl, optionally substituted C₁-C₆ alkinyl, optionally substituted C₃-C₈ cycloalkyl, NR₆R₇, OR₆, SR₆, wherein R₆ and R₇ are independently selected from hydrogen, optionally substituted C₃-C₈ cycloalkyl, optionally substituted aryl, optionally substituted 5- or 6-membered heterocycle, (CH₂)ₙC(O)OR₈, (CH₂)ₙR₉, wherein n, R₈ and R₉ are as defined above;
R₃ is hydroxyl, halogen, NH₂, NO₂, cyano, SH, optionally substituted C₁-C₆ alkyl, optionally substituted C₃-C₈ cycloalkyl, optionally substituted aryl, optionally substituted 5- or 6-membered heterocycle,
for the manufacture of a medicament for the treatment of a hyperproliferative and/or angiogenesis related disease.

2. The use of anyone of the preceding claims, wherein the disease is a (hyper)proliferative disease.

3. The use of claim 2, wherein the disease is a benign or malignant tumour.

4. The use of claim 2, wherein the disease is a benign or malignant neoplasia, including for example, but not exclusively, tumours of the brain, kidney, liver, adrenal gland, bladder, breast, stomach, ovaries, rectum, prostate pancreas, lung, vagina, thyroid, connective tissue (sarcoma), gastrointestinal tract, tumours of the neck and head, tumours derived from cells of the hematopietic system (including leukemias, lymphomas and multiple myeloma), epidermal hyperproliferation, including for example, but not exclusively, prostate hyperplasia.

5. The use of claim 4, wherein the disease is a gastric tumour, colon carcinoma, colorectal adenoma, psoriasis, mammary carcinoma or leukaemia.

6. The use of anyone of claims 1, wherein the disease is related to angiogenesis.

7. The use of anyone of claims 1or 6, wherein the disease is psoriasis, Kaposi's sarcoma, restenosis, endometriosis, Crohn's disease, leukaemia, arthritis, rheumatoid arthritis, hemangioma, angiofibroma, diabetic retinopathy, age-related macular degeneration, neovascular glaucoma, renal diseases,
glomerulonephritis, diabetic nephropathy, malignant nephrosclerosis, thrombotic microangiopathic syndromes, transplant rejections, glomerulopathy, cirrhosis of the liver, mesangial cell-proliferative diseases, arteriosclerosis, injuries of the nerve tissue.

8. The use of claim 6 for inhibiting the re-occlusion of vessels after balloon catheter treatment, for holding vessels open in vascular prosthetics or after inserting mechanical devices, such as, e.g., stents, as immunosuppressants, as an aid in scar-free wound healing, and for treating dermatitis.

9. The use of anyone of the preceding claims, wherein the kinase inhibitor is an inhibitor of a member of the ephrin (Eph) family of receptor tyrosine kinases.

10. The use of claim 9, wherein the kinase is an ephrin B2 or ephrin B4 receptor tyrosine kinase.

11. The use of a kinase inhibitor of anyone of claims 1, having formula Ia 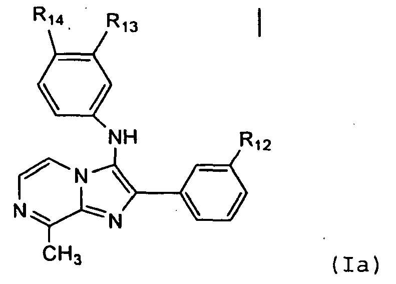 wherein R12 is hydrogen or methoxy and R13 and R14 are independently selected from hydrogen, methyl, halogen.

12. The use of claim 11, wherein
R12 is hydrogen and either R13 or R14 is H or
R13 is methyl, Cl, Br, or F or
R12 is hydrogen, and R13 and R14 are both either F or Cl or
R12 is methoxy, R13 is methyl and R14 is hydrogen or
R12 is methoxy, R13 is hydrogen and R14 is C1 or
R12 is methoxy, R13 is C1 and R14 is hydrogen or
R12 is methoxy, R13 is methyl and R14 is hydrogen.

13. The compounds of the formula Ia as defined in anyone of claims 11-12 as a pharmaceutical.

14. The compounds as defined in anyone of claims 11-12.

15. A pharmaceutical composition comprising a compound as defined in anyone of claims 11-12.

16. A pharmaceutical composition comprising a compound as defined in anyone of claims 11-12 and an acceptable pharmaceutical carrier.

17. Use of a compound as defined in anyone of claims 11-12 as research tool in functional genomics, excluding methods for treatment of the human or animal body by surgery or therapy and diagnostic methods practiced on the human or animal body.

18. Use of a compound as defined in anyone of claims 11-12 as research tool in drug discovery, excluding methods for treatment of the human or animal body by surgery or therapy and diagnostic methods practiced on the human or animal body.

19. Use of a compound as defined in anyone of claims 11-12 as research tool in target validation, excluding methods for treatment of the human or animal body by surgery or therapy and diagnostic methods practiced on the human or animal body.

20. Use of a compound as defined in anyone of claims 11-12 as research tool in ex vivo diagnostics.

## Patentansprüche

1. Verwendung eines Kinase Inhibitors der Formel (I) 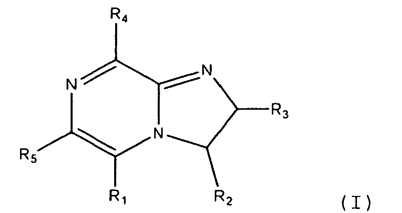 worin
R₁ und R₅ unabhängig voneinander ausgewählt sind aus Wasserstoff, Halogen, Nitro, CF₃,
gegebenenfalls substituiertem linearem oder verzweigtem C₁-C₆ Alkyl,
gegebenenfalls substituiertem C₁-C₆ Alkenyl,
gegebenenfalls substituiertem C₁-C₆ Alkinyl,
gegebenenfalls substituiertem C₃-C₈ Cycloalkyl, alkoxy, NR₆R₇, OR₆, SR₆, (CH2)ₙCHR₆R₇,
worin n 0, 1, 2, 3, 4 ist und R₆ und R₇ unabhängig voneinander ausgewählt sind aus Wasserstoff, gegebenenfalls substituiertem C₃-C₈ Cycloalkyl, gegebenenfalls substituiertem Aryl, gegebenenfalls substituiertem 5- oder 6- gliedrigem Heterocyclus,
(CH₂)ₙCO(O)R₈, (CH₂)ₙR₉.
worin n wie oben definiert ist, R₈ Wasserstoff, gegebenenfalls substituiertes C₁-C₆ Alkyl, gegebenenfalls substituiertem C₃-C₈ Cycloalkyl, gegebenenfalls substituiertem Aryl ist, R9 ausgewählt ist aus Wasserstoff, C₁-C₆ Alkoxy, gegebenenfalls substituiertem C₃-C₈ Cycloalkyl, gegebenenfalls substituiertem Aryl, Halogen, Hydroxyl, NO₂, NH₂, SO₂NH₂, Cyano,
und wobei alle optionalen Substituenten ausgewählt sind aus der Gruppe von Methyl, Methoxy, Halogen, OH, NO₂ NH₂, -C(O)OMe, cyano
R₂ und R₄ unabhängig voneinander ausgewählt sind aus Wasserstoff,
gegebenenfalls substituiertem linearem oder verzweigtem C₁-C₆ Alkyl,
gegebenenfalls substituiertem C₁-C₆ Alkenyl,
gegebenenfalls substituiertem C₁-C₆ Alkinyl,
gegebenenfalls substituiertem C₃-C₈ Cycloalkyl,
NR₆R₇, OR₆, SR₆,
worin R₆ und R₇ unabhängig voneinander ausgewählt sind aus Wasserstoff, gegebenenfalls substituiertem C₃-C₈ Cycloalkyl, gegebenenfalls substituiertem Aryl, gegebenenfalls substituiertem 5- oder 6- gliedrigem Heterocyclus,
(CH₂)ₙCO(O)R₈, (CH₂)ₙR9,
worin n, R₈ und R₉ wie oben definiert ist;
R₃ Hydroxy, Halogen, NH₂, NO₂, Cyano, SH, gegebenenfalls substituiertes C₁-C₆ Alkyl, gegebenenfalls substituiertes C₃-C₈ Cycloalkyl, gegebenenfalls substituiertes Aryl, gegebenenfalls substituierter 5- oder 6-gliedriger Heterocyclus ist;
zur Herstellung eines Medikamentes zur Behandlung einer hyperproliferativen und / oder angiogenese - verknüpften Erkrankung.

2. Die Verwendung nach einem der vorgängigen Ansprüche, wobei die Erkrankung eine (hyper)proliferative Erkrankung ist.

3. Die Verwendung gemäss Anspruch 2, wobei die Krankheit ein gutartiger oder bösartiger Tumor ist.

4. Die Verwendung gemäss Anspruch 2, wobei die Krankheit ein gutartige oder bösartige Neoplasie, umfassend beispielsweise, aber nicht ausschliesslich, Tumore des Hinrs, der Niere, der Nebenniere, der Leber, der Blase, der Brust, des Magens, der Eierstöcke, des Mastdarms, der Prostata, der Bauchspeicheldrüse, der Lunge, der Vagina, der Schilddrüse, des Bindegewebes (Sarcoma), des Magen - Darm - Traktes,
Tumore an Nacken und Kopf,
Tumore abgeleitet von Zellen des hemopatischen Systems (umfasend Leukämie, Lymphoma, multiple Myeloma),
epidermale Hyperproliferation, umfassend beispielsweise, aber nicht ausschliesslich, Prostata Hyperplasia.

5. Die Verwendung gemäss Anspruch 4, wobei die Krankheit ein Magenkrebs, Dickdarmkarzinom, kolorektales Adenom, Psoriasis, Brustdrüsen Karzinom oder Leukämie ist.

6. Die Verwendung gemäss Anspruch 1, wobei die Krankheit zu Angiogenese im Bezug steht.

7. Die Verwendung gemäss Anspruch 1 oer 6, wobei die Krankheit Psoriasis, Koposi's Sarkom, Restenose, Endometiose, Crohn's Krankheit, Leukämie, Arthiritis, rheumatische Arthritis, Hämangiom, Angiofibrom, diabetische Rhetionpatie, alters bedingte Makuladegeneration, neovaskulares Glaukom, Nierenkrankheit, Glomerulonephritis, disbetisches Nierenleiden, bösartige Nephrosklerose, thrombotisches mikorangiopatisches Syndrom, Transplantat Abstossung, Glomerulopathie, Leberzerrung, mesangiale Zell-proliferative Krankheit, Arteriosklerose, Verletzung des Nervengewebes ist.

8. Die Verwendung gemäss Anspruch 6 zur Inhibierung des Widerverschlusses von Gefässen nach einer Ballon Katheder Behandlung, zum Offenhalten von Gefässen in vascularer Prothetik, oder nach dem einführen von mechanischen Vorrichtungen wie beispielsweise *stents,* als Immunosuppresant, als ein Hilfsmittel beim Narbenfreien Wundheilen, und zur Behandlung von entzündlichen Hautreaktionen.

9. Die Verwendung gemäss irgendeinem der vorgängigen Ansprüche, wobei der Kinase Inhibitor ein Mitglied der Ephrin (Eph) Familie der Rezeptor Tyrosin Kinasen ist.

10. Die Verwendung gemäss Anspruch 9, wobei die Kinase eine Ephrin B2 oder Ephrin B4 Rezeptor Tyrosin Kinase ist.

11. Die Verwendung eines Kinase Inhibitors gemäss Anspruch 1, welcher die Formel Ia hat, 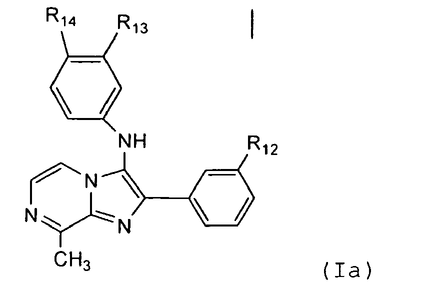 worin R₁₂ Wasserstoff oder Methoxy und R₁₃ und R₁₄ unabhängig voneinander ausgewählt sind aus Wasserstoff, Methyl, Halogen.

12. Die Verwendung gemäss Anspruch 11, worin
R₁₂ Wasserstoff ist und entweder R₁₃ oder R₁₄ Wasserstoff ist oder R₁₃ Methyl, Chlor, Brom oder Fluor ist, oder
R₁₂ Wasserstoff ist und R₁₃ und R₁₄ sind beide entweder Fluor oder Chlor oder
R₁₂ Methoxy ist, R₁₃ Methyl ist und R₁₄ Wasserstoff ist, oder
R₁₂ Methoxy ist, R₁₃ Wasserstoff ist und R₁₄ Chlor ist, oder
R₁₂ Methoxy ist, R₁₃ Chlor ist und R₁₄ Wasserstoff ist, oder
R₁₂ Methoxy ist, R₁₃ Methyl ist und R₁₄ Wasserstoff ist.

13. Die Verbindungen der Formel Ia gemäss einem der Ansprüche 11 - 12 als Pharmazeutikum.

14. Die Verbindungen der Formel Ia gemäss einem der Ansprüche 11 - 12.

15. Eine Pharmazeutische Zusammensetzung umfassend eine Verbindung gemäss einem der Ansprüche 11 - 12.

16. Eine Pharmazeutische Zusammensetzung umfassend eine Verbindung gemäss einem der Ansprüche 11 - 12 und einen akzeptablen pharmazeutischen Träger.

17. Die Verwendung einer Verbindung gemäss einem der Ansprüche 11 - 12 als Forschungswerkzug in der funktionalen Genomik, unter Ausschluss von Verfahren zur chirurgischen oder therapeutischen Behandlung des menschlichen oder tierischen Körpers sowie Diagnostizierverfahren die am menschlichen oder tierischen Körper vorgenommen werden.

18. Die Verwendung einer Verbindung gemäss einem der Ansprüche 11 - 12 als Forschungswerkzug in der Auffindung von Arzneimitteln, unter Ausschluss von Verfahren zur chirurgischen oder therapeutischen Behandlung des menschlichen oder tierischen Körpers sowie Diagnostizierverfahren die am menschlichen oder tierischen Körper vorgenommen werden.

19. Die Verwendung einer Verbindung gemäss einem der Ansprüche 11 - 12 als Forschungswerkzug in der Valdierung von Zielmolekülen, unter Ausschluss von Verfahren zur chirurgischen oder therapeutischen Behandlung des menschlichen oder tierischen Körpers sowie Diagnostizierverfahren die am menschlichen oder tierischen Körper vorgenommen werden.

20. Verwendung einer Verbindung gemäss einem der Ansprüche 11 - 12 als Forschungswerkzeug in der *ex vivo* Diagnose.

## Revendications

1. Utilisation d'un inhibiteur de kinase de formule I 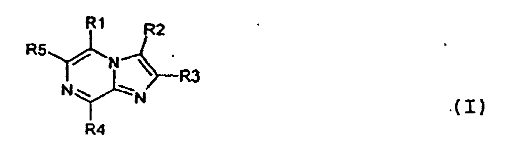 dans laquelle
R1 et R5 sont choisis indépendamment parmi hydrogène, halogène, cyano, nitro, CF3, alcoyle linéaire ou ramifié substitué éventuellement ayant de 1 à 6 atomes de carbone, alcényle substitué éventuellement ayant de 1 à 6 atomes de carbone, cycloalcoyle substitué éventuellement ayant de 3 à 8 atomes de carbone, alcoxy, NR₆R₇, OR₆, SR₆, (CH₂)ₙCHR₆R₇, dans laquelle n est 0, 1, 2, 3, 4 et R₆ et R₇ sont choisis indépendamment parmi hydrogène, cycloalcoyle substitué éventuellement ayant de 3 à 8 atomes de carbone, aryle substitué éventuellement à 5 ou 6 chaînons, (CH₂)ₙC(O)OR₈, (CH₂)ₙR₉, dans lesquelles n est tel que défini ci-dessus, R₈ est hydrogène, alcoyle substitué éventuellement ayant de 1 à 6 atomes de carbone, cycloalcoyle substitué éventuellement ayant de 3 à 8 atomes de carbone, aryle substitué éventuellement et R₉ est choisi parmi hydrogène, alcoxy ayant de 1 à 6 atomes de carbone, cycloalcoyle substitué éventuellement ayant de 3 à 8 atomes de carbone, aryle substitué éventuellement, halogène, hydroxyle, NO_{2,} NH₂, SO₂NH, cyano et dans laquelle tous les substituant éventuels sont choisis dans le groupe de méthyle, méthoxy, halogène, OH, NO₂, NH₂, -C(O)OMe, cyano ;
R₂ et R₄ sont choisis indépendamment parmi hydrogène, alcoyle linéaire ou ramifié substitué éventuellement ayant de 1 à 6 atomes de carbone, alcényle substitué éventuellement ayant de 1 à 6 atomes de carbone, alcényle substitué éventuellement ayant de 1 à 6 atomes de carbone, cycloalcoyle substitué éventuellement ayant de 3 à 8 atomes de carbone, NR₆R₇, OR₆, SR₆, R₆ et R₇ étant choisis indépendamment parmi hydrogène, cycloalcoyle substitué éventuellement ayant de 3 à 8 atomes de carbone, aryle substitué éventuellement, hétérocycle substitué éventuellement à 5 ou 6 chaînons (CH₂)ₙC(O)OR₈, (CH₂₎ₙR₉, dans lesquelles n, R8 et R9 sont comme définis ci-dessus ;
R3 est hydroxyle, halogène, NH₂, NO₂, cyano, SH, alcoyle substitué éventuellement ayant de 1 à 6 atomes de carbone, cycloalcoyle substitué éventuellement ayant de 3 à 8 atomes de carbone, aryle substitué éventuellement, hétérocyle substitué éventuellement à 5 ou 6 chaînons,
pour la fabrication d'un médicament pour le traitement d'une maladie hyperproliférative et/ou en relation avec l'angiogénèse.

2. Utilisation suivant l'une quelconque des revendications précédentes, dans laquelle la maladie est une maladie (hyper)proliférative.

3. Utilisation suivant la revendication 2, dans laquelle la maladie est une tumeur bénigne ou maligne.

4. Utilisation suivant la revendication 2, dans laquelle la maladie est une néoplasie bénigne ou maligne, y compris par exemple, mais non exclusivement, une tumeur du cerveau, du rein, du foie, de la glande surrénale, de la vessie, du sein, de l'estomac, des ovaires, du rectum, de la prostate, du pancréas, du poumon, du vagin, de la thyroïde, du tissu conjonctif (sarcome), du tractus gastro-intestinal, des tumeurs du cou et de la tête, des tumeurs dérivées de cellules du système hématopiétique (comprenant des leucémies, des lymphomes et des myélomes multiples), une hyperprolifération épidermique comprenant par exemple sans exclusion l'hyperplasie de la prostate.

5. Utilisation suivant la revendication 4, dans lequel la maladie est une tumeur gastrique, un carcinome du colon, un adénome colorectal, un psoriasis, un carcinome mammaire ou une leucémie.

6. Utilisation suivant l'une quelconque des revendications 1, dans laquelle la maladie est en relation avec l'angiogénèse.

7. Utilisation suivant l'une quelconque des revendications 1 ou 6, dans laquelle la maladie est le psoriasis, le sarcome de Kaposi, la resténos, l'endométriose, la maladie de Crown, la leucémie, la polyarthrite rhumatoïde, l'hémangiome, l'angiofibrome, la rétinopathie diabétique, la dégénération maculaire en liaison avec la vieillesse, la dégénération, le glaucome vasculaire, les maladies rénales, la glomérulonéphrite, la néphropathie diabétique, la néphrosclérose maligne, les syndromes thrombotiques microangiopathiques, les rejets de transplantation, la glomérulopathie, la cirrhose du foie,les maladies mésangiales à prolifération de cellules, l'artériosclérose, les lésions du tissu nerveux.

8. Utilisation suivant la revendication 6 pour inhiber la réocclusion de vaisseaux après un traitement par cathéter à ballon pour maintenir les vaisseaux ouverts dans des prothèses vasculaires ou après insertion de dispositifs mécaniques, tels que, par exemple, des endoprothèses comme immunosuppresseurs, comme adjuvant de guérison de blessure sans cicatrice et pour traiter l'eczéma.

9. Utilisation suivant l'une quelconques des revendications précédentes, dans laquelle l'inhibiteur de kinase est un inhibiteur de la famille de l'éphrine (Eph) des kinases réceptrices de la tyrosine.

10. Utilisation suivant la revendication 9, dans laquelle la kinase est une kinase réceptrice de la tyrosine éphrine B2 ou éphrine B4.

11. Utilisation suivant l'une quelconque de la revendication 1 ayant la formule la 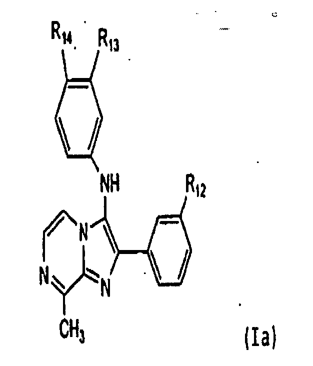 dans laquelle R12 est hydrogène ou méthoxy et R13 et R14 sont choisis indépendamment parmi hydrogène, méthyle, halogène.

12. Utilisation suivant la revendication 11, dans laquelle R12 est hydrogène et soit R13, soit R14 est H ou
R13 est méthyle, Cl, Br, ou F ou
R12 est hydrogène et R13 et R14 sont tous deux soit F soit Cl ou
R12 est méthoxy, R13 est méthyle et R14 est hydrogène ou
R12 est méthoxy, R13 est hydrogène et R14 est Cl ou
R12 est méthoxy, R13 est Cl et R14 est hydrogène ou
R12 est méthoxy, R13 est méthyle et R14 est hydrogène.

13. Composés de formule la tels que définis dans l'une quelconque des revendications 11 à 12 comme médicament.

14. Composés de formule la tels que définis dans l'une quelconque des revendications 11 à 12.

15. Composition pharmaceutique comprenant un composé tel que défini dans l'une quelconque des revendications 11 et 12.

16. Composition pharmaceutique comprenant un composé tel que défini dans l'une quelconque des revendications 11 et 12 et un véhicule acceptable pharmaceutiquement.

17. Utilisation d'un composé tel que défini à l'une quelconque des revendications 11 et 12 comme outil de recherche en génomique fonctionnelle, à l'exclusion de procédés de traitement du corps humain ou animal par chirurgie ou thérapie et de procédés de diagnostic pratiqués sur le corps humain ou animal.

18. Utilisation d'un composé tel que défini à l'une quelconque des revendications 11 et 12 comme outil de recherche de médicaments, à l'exclusion de procédés de traitement du corps humain ou animal par chirurgie ou thérapie et de procédés de diagnostic pratiqués sur le corps humain ou animal.

19. Utilisation d'un composé tel que défini à l'une quelconque des revendications 11 et 12 comme outil de recherche dans une validation de cibles, à l'exclusion de procédés de traitement du corps humain ou animal par chirurgie ou thérapie et de procédés de diagnostic pratiqués sur le corps humain ou animal.

20. Utilisation d'un composé tel que défini dans l'une quelconque des revendications 11 et 12 comme outil de recherche dans des diagnostics ex vivo.
